# EUROPEAN PATENT APPLICATION

(11) **EP 4 585 615 A1**
(43) Date of publication of application: **16.07.2025**
(21) Application number: 22957767.1
(22) Date of filing: 09.09.2022
(51) Int. Cl.: C07K 16/28, C07K 16/30, C07K 16/46, C12N 5/10, A61K 39/395, A61P 35/00

(54) **MULTISPECIFIC ANTIBODY BINDING TO BCMA, GPRC5D AND CD3, AND USE THEREOF**

(71) Applicant: Beijing Mabworks Biotech Co., Ltd, Beijing 100176 (CN); Kyinno Biotechnology Co., Ltd., Beijing 101111 (CN)
(72) Inventor: PENG, Hao, Beijing 101111 (CN); LI, Jiangmei, Beijing 100176 (CN); WU, Guojin, Beijing 101111 (CN); HAO, Feng, Beijing 101111 (CN); HU, Wenqi, Beijing 100176 (CN); NING, Jinying, Beijing 101111 (CN); LI, Feng, Beijing 100176 (CN)
(74) Representative: Krauss, Jan
(86) International application number: PCT/CN2022/117979
(87) International publication number: WO 2024/050797

(57) **Abstract**

The present application relates to a multi-specific antibody that simultaneously targets BCMA, GPRC5D and CD3, and its use in treatment of diseases such as tumors.

## Description

### FIELD OF THE INVENTION

The present disclosure relates to a multi-specific antibody simultaneously targeting BCMA, GPRC5D and CD3, and its use in treatment of diseases such as tumors.

### BACKGROUND OF THE INVENTION

Multiple myeloma (MM) is a disease with malignant proliferation of plasma cells, which is characterized by the unlimited growth of the plasma cells in bone marrow, just like the tumor cells do, along with secretion of monoclonal immunoglobulins in most cases, eventually causing organ or tissue damage. For example, MM is often accompanied with multiple osteolytic lesions, hypercalcemia, anemia, kidney damage and etc. MM is common for middle-aged and older adults, and there is a clear sex disparity in incidence with more male patients than the female ones. MM accounts for 10-15% of hematologic malignancies, and is the third common hematologic malignancy following leukemia and lymphoma. The incidence is 9 per 100,000 in the US and about 1 per 100,000 in China, and has been increasing over the years.

In recent years, in despite of the advances in MM treatment involving chemotherapies, protease inhibitors, immunomodulators and CD38 targeting antibodies, a substantial portion of patients are not responsive to these therapies, or respond to the therapies for only a short time. Further, MM is almost incurable, the patients gradually become resistant to the therapies, causing recurrence. Therefore, there is an urgent need for new medicaments in the field.

The antigens currently known for MM targeted therapies are very limited, and BCMA and GPRC5D are two of them.

### BCMA and BCMA-targeted therapies

B cell maturation antigen, also referred to as BCMA, CD269 or TNFRSF17, is a type I transmembrane protein and a member of the tumor necrosis factor receptor (TNFR) superfamily. BCMA, along with BAFF-receptor (BAFFR) and transmembrane activator and calcium modulator and cyclophilin ligand interactor (TACI), the members of the same TNFR superfamily, plays important roles in survival of B cells in different development stages (Rickert R.C et al., (2011) Immunological Reviews 244(1):115-133). BCMA is mainly expressed on the surfaces of mature B lymphocytes and plasma cells, with a few expression in hematopoietic stem cells or nonhematopoietic tissues. Its ligands include B cell activating factor (BAFF) and A proliferation-inducing ligand (APRIL), of which the latter one has higher BCMA binding affinity.

BCMA overexpression has been found on the surfaces of the malignant plasma cells in the bone marrow of MM patients. BCMA promotes survival of malignant plasma cells in the bone marrow, and the APRIL-BCMA signaling in the malignant plasma cells promotes malignant tumor cells' proliferation, evasion from apoptosis, and production of potent immune-inhibitory molecules such as IL-10, PD-L1 and TGF-β (Tai Y-T et al., (2016) Blood. 127(25):3225-3236). BCMA overexpression and activation were found to be associated with MM progression in many animal models and human patients (Tai Y-T *et al.,* (2016) *Supra;* Sanchez E et al., (2016) Clin Cancer Res. 22:3383-3397).

Due to BCMA's selective expression/distribution pattern, i.e., widely present on MM cell surfaces but with very low to no expression on cells in normal tissues, and BCMA's relatively long serum half-life, it has become an ideal therapeutic target for MM and other hematological malignancies. Compared to the CD138 molecules which are uniquely expressed by the plasma cells but disappear rapidly from the cell surfaces, BCMA is evidently a better biomarker for MM's malignant plasma cells.

### GPRC5D and GPRC5D-targeted therapies

GPRC5D, i.e., G protein-coupled receptor class C group 5 member D, is a type C 7-pass transmembrane receptor protein, with its ligand(s) and signaling mechanisms remains to be elucidated.

GPRC5D was found to be a marker for MM in the test of bone marrow samples from patients with MM, acute leukemia and diffuse large B-cell lymphoma (Cohen Y et al., (2013) Hematology 18(6):348-351). This protein is mainly expressed in cells with a plasma cell phenotype, including most malignant bone marrow plasma cells, and its expression pattern in CD138⁺ MM cells from bone marrow samples is quite similar to that of BCMA. The GPRC5D protein expression in normal tissues is limited to hair follicle (Smith EL et al., (2019) Sci Transl Med. 11(485):eaau7746). In addition, the GPRC5D mRNA expression levels in MM patients correlate with genetic aberrations such as Rb-1 deletion and poor prognosis (Atamaniuk J et al., (2012) Eur J Clin Invest. 42(9):953-960).

Such selective expression pattern makes GPRC5D a promising therapeutic target for plasma cell related diseases such as MM. JNJ-64407564, a bi-specific antibody against this target, is currently in clinical trials.

### T cells and CD3

The CD3 molecule is a marker on T cell surfaces. It forms a TCR-CD3 complex with a T cell receptor (TCR), to play key roles in antigen recognition and immune signal transduction. A TCR molecule consists of an α chain and a β chain, or a γ chain and a δ chain. The intracellular domain of each chain does not have signal transduction function, so the intracellular signaling within the T cells totally depends on the CD3 protein. A CD3 molecule is composed of a γ chain, a δ chain, two ε chains, and two ζ chains, forming three dimers, εγ, εδ and ζζ, in the TCR/CD3 complex. The ε, γ and δ chains are all type I transmembrane proteins, each with an immunoglobulin-like extracellular domain. The ε, γ, δ and ζ chains contain 10 immunoreceptor tyrosine-based activation motifs (ITAMs) in total in the intracellular domains, and the phosphorylation of ITAM enables these chains bind to ZAP70, transducing T cell activation signals to downstream elements.

A CD3 targeted antibody may form a bi-specific molecule with a functional moiety targeting a disease associated antigen (such as a tumor associated antigen),which physically links T cells with the disease associated antigens, resulting in T cell activation and T cell-mediated death of disease associated cells. For instance, a bi-specific molecule targeting CD3 and a tumor associated antigen may pull T cells to the tumor cells, such that the T cells are activated to release supramolecular attack particles (SMAPs) containing more than 280 proteins. The SMAP comprises granzymes and perforins in the core region, where the perforins can form pores on the plasma membrane of tumor cells and the granzymes can induce tumor cell apoptosis (Š. Bálint *et al.,* (2020) Science 368(6493): 897-901).

CD3 molecules may aggregate on T cell surfaces by the action of the anti-CD3 antibodies, simulating the process where TCR recognizes the MHC-antigen peptide, so as to trigger the TCR complex's signaling in T cells, releasing cytokines such as IL-2, IFN-γ and TNF-α, which promote T cell proliferation and differentiation. T cell proliferation and differentiation is a mixed blessing in tumor treatment, which on one side produces more T cells for killing tumor cells, but on the other side causes severe toxicities, i.e., cytokine release syndrome ("CRS"), in subjects. Clinically, side effects associated with CRS include not only fatigue, vomiting, tachycardia, hypertension, and back pain, but also central nervous system (CNS) reactions such as seizures, encephalopathy, cerebral edema, aseptic meningitis, and headache. For example, CRS was observed in the therapies using anti-CD3 monospecific antibodies such as OKT3, which is believed to be caused by cross-linking of the antibodies through their binding to Fc receptors (FcRs) inside the body (Herold KC et al., (2003) J Clin Invest. 111(3):409-418). Therefore, in subsequent antibody development, the Fc regions of anti-CD3 antibodies such as Teplizumab were engineered to present weak FcR binding capability.

Similarly, CRS is inevitable in the therapies using CD3 targeted bi-specific or multi-specific antibodies. For example, in the clinical trials of blinatumomab, a bispecific CD19/CD3 T cell engager antibody, severe CRS and CNS toxicity were frequently observed. The modification to the Fc regions, as made to the monospecific anti-CD3 antibodies, is insufficient to alleviate the bi-specific anti-CD3 antibody-mediated CRS, as the binding of the antigen associated antigen-targeted functional moiety in the bi-specific antibodies to a target cell renders antibody cross-linking, inducing abundant cytokine release by T cells.

### Tri-specific antibody targeting GPRC5D, BCMA and CD3

BCMA targeting is promising in MM treatment. However, due to BCMA expression heterogeneity, not all tumor cells express BCMA, even in the tumor samples from the same patient, resulting in different treatment outcomes. Further, the membrane-bound BCMA may be shed off the cell surface by the action of the γ-secretase, causing the change of cell surface BCMA levels (Brudno JN et al., (2018) J. Clin. Oncol 36(22):2267-2280; Laurent SA et al., (2015) Nat Commun. 6:7333). These problems may be solved to some extent by targeting another marker such as GPRC5D or FcRH5 on MM cells. However, the expression distribution of BCMA is somewhat similar to that of GPRC5D, it is uncertain whether targeting them both can result in more durable and stable MM tumor cell binding. In addition, an antigen binding domain against CD3 may be added to such a bi-specific antibody, inducing recruitment of T cells around MM cells in the patients, to activate T cells to kill MM cells. In the meanwhile, as mentioned above, attention should be paid to the toxicities caused by a bi- or multi-specific anti-CD3 antibody. In the first half of Year 2021, the clinical trials of several BCMA×CD3 bi-specific antibodies (AMG701, Elranatamab and etc.) were suspended for safety issues.

Citation or identification of any document in this application is not an admission that such document is available as prior art to the present invention.

### SUMMARY OF THE INVENTION

The inventors of the application, constructed a tri-specific GPRC5D × BCMA × CD3 antibody with elegant design that possesses potent anti-tumor activity and induces relatively low toxicity, surmounting and ameliorating the efficacy-toxicity-balance problem of the bi-specific anti-CD3 antibodies currently used in MM treatment, including the BCMA × CD3 bi-specific antibodies and the GPRC5D × CD3 bi-specific antibodies. The construction of such a multi-specific antibody, was by no means a simple and random combination of various binding moieties, the interactions among binding moieties were taken into consideration. During construction of the antibody of the present disclosure, the tumor specific antigen species on MM cells to be bound was adjusted and optimized, to avoid tumor evasion from killing due to tumor heterogeneity, and the CD3 signaling activation-mediated cytokine release was reduced, to get a better therapeutic window.

Therefore, in a first aspect, the present application provides a multi-specific antibody, which may comprise: i) an antigen binding domain against CD3, ii) an antigen binding domain against BCMA, and iii) an antigen binding domain against GPRC5D.

The antigen binding domain against CD3 may be an agonistic antigen binding domain, which may specifically bind CD3 and activate CD3 signaling. In particular, the antigen binding domain against CD3 does not bind to CD3δ or CD3ε alone, and binds to the CD3δ&ε complex only. The antigen binding domain against CD3, has almost no CD3 signaling activation effect, i.e., almost does not activate T cells, when present as a free molecule, but possesses CD3 signaling activation ability only if the other two antigen binding domains in the multi-specific antibody bind to their respective antigens to enable antibody "cross-linking". In certain embodiments, the antigen binding domain against CD3 is an antibody or an antigen binding portion thereof that specifically binds CD3.

The antigen binding domain against BCMA may bind BCMA and optionally block BCMA-APRIL binding/interaction, i.e., may optionally be an antagonistic antigen binding domain. In particular, the antigen binding domain against BCMA may bind to BCMA on target cells, hardly affected by the soluble BCMAs in the environment. In certain embodiments, the antigen binding domain against BCMA is an antibody or an antigen binding domain thereof that specifically binds BCMA.

The antigen binding domain against GPRC5D may specifically bind GPRC5D. In certain embodiments, the antigen binding domain against GPRC5D may be an antibody or an antigen binding portion thereof that specifically binds GPRC5D.

The multi-specific antibody of the disclosure may comprise e.g., one antigen binding domain against CD3, one antigen binding domain against BCMA, and one antigen binding domain against GPRC5D.

The multi-specific antibody of the disclosure may be an IgG-like antibody, comprising an antigen binding domain against CD3 in the Fab or Fv format, an antigen binding domain against GPRC5D in the Fab or Fv format, and an antigen binding domain against BCMA in the single chain fragment variable (scFv) format.

In certain embodiments, the multi-specific antibody may comprise i) an anti-CD3 half-antibody, comprising a heavy chain variable region, a heavy chain constant region, a light chain variable region, and an optional light chain constant region, ii) an anti-GPRC5D half-antibody, comprising a heavy chain variable region, a heavy chain constant region, a light chain variable region, and an optional light chain constant region, and iii) an antigen binding domain against BCMA in the scFv format, comprising a heavy chain variable region, an optional first linker, and a light chain variable region, wherein the anti-CD3 half-antibody and the anti-GPRC5D half-antibody may form an IgG full-length antibody, and the antigen binding domain against BCMA in the scFv format may be linked, optionally via a second linker, to N-terminus of the heavy chain variable region, N-terminus of the light chain variable region, C-terminus of the heavy chain constant region or C-terminus of the light chain constant region of the anti-CD3 half-antibody. In certain embodiments, the antigen binding domain against BCMA in the scFv format may be linked to N-terminus of the heavy chain variable region or N-terminus of the light chain variable region of the anti-CD3 half-antibody.

The multi-specific antibody of the disclosure may comprise:
i) a first polypeptide chain, comprising a heavy chain variable region specifically binding BCMA, a light chain variable region specifically binding BCMA, a heavy chain variable region specifically binding CD3, and a heavy chain constant region,
ii) a second polypeptide chain, comprising a light chain variable region specifically binding CD3,
iii) a third polypeptide chain, comprising a heavy chain variable region specifically binding GPRC5D, and a heavy chain constant region, and
iv) a fourth polypeptide chain, comprising a light chain variable region specifically binding GPRC5D; or alternatively,

i) a first polypeptide chain, comprising a heavy chain variable region specifically binding CD3, and a heavy chain constant region,
ii) a second polypeptide chain, comprising a heavy chain variable region specifically binding BCMA, a light chain variable region specifically binding BCMA, and a light chain variable region specifically binding CD3,
iii) a third polypeptide chain, comprising a heavy chain variable region specifically binding GPRC5D, and a heavy chain constant region, and
iv) a fourth polypeptide chain, comprising a light chain variable region specifically binding GPRC5D,
wherein the heavy chain variable region specifically binding BCMA and the light chain variable region specifically binding BCMA form the antigen binding domain against BCMA, the heavy chain variable region specifically binding CD3 in the first polypeptide chain and the light chain variable region specifically binding CD3 in the second polypeptide chain form the antigen binding domain against CD3, the heavy chain variable region specifically binding GPRC5D in the third polypeptide chain and the light chain variable region specifically binding GPRC5D in the fourth polypeptide chain form the antigen binding domain against GPRC5D, and the heavy chain constant region in the first polypeptide chain and the heavy chain constant region in the third polypeptide chain are associated together.

The first polypeptide chain may comprise, from N-terminus to C-terminus, the heavy chain variable region specifically binding BCMA, the light chain variable region specifically binding BCMA, the heavy chain variable region specifically binding CD3, and the heavy chain constant region; or from N-terminus to C-terminus, the light chain variable region specifically binding BCMA, the heavy chain variable region specifically binding BCMA, the heavy chain variable region specifically binding CD3, and the heavy chain constant region. The third polypeptide chain may comprise, from N-terminus to C-terminus, the heavy chain variable region specifically binding GPRC5D, and the heavy chain constant region.

In certain embodiments, the multi-specific antibody may comprise:
i) a first polypeptide chain, comprising, from N-terminus to C-terminus, a heavy chain variable region specifically binding BCMA, a first linker, a light chain variable region specifically binding BCMA, a second linker, a heavy chain variable region specifically binding CD3, and a heavy chain constant region; or alternatively a light chain variable region specifically binding BCMA, a first linker, a heavy chain variable region specifically binding BCMA, a second linker, a heavy chain variable region specifically binding CD3, and a heavy chain constant region,
ii) a second polypeptide chain, comprising, from N-terminus to C-terminus, a light chain variable region specifically binding CD3 and a light chain constant region,
iii) a third polypeptide chain, comprising, from N-terminus to C-terminus, a heavy chain variable region specifically binding GPRC5D, and a heavy chain constant region, and
iv) a fourth polypeptide chain, comprising, from N-terminus to C-terminus, a light chain variable region specifically binding GPRC5D, and a light chain constant region.

Alternatively, the first polypeptide chain may comprise, from N-terminus to C-terminus, a heavy chain variable region specifically binding CD3, and a heavy chain constant region. The second polypeptide chain may comprise, from N-terminus to C-terminus, a heavy chain variable region specifically binding BCMA, a light chain variable region specifically binding BCMA, and a light chain variable region specifically binding CD3, or from N-terminus to C-terminus, a light chain variable region specifically binding BCMA, a heavy chain variable region specifically binding BCMA, and a light chain variable region specifically binding CD3. The third polypeptide chain may comprise, from N-terminus to C-terminus, a heavy chain variable region specifically binding GPRC5D, and a heavy chain constant region.

In certain embodiments, the multi-specific antibody may comprise:
i) a first polypeptide chain, comprising, from N-terminus to C-terminus, a heavy chain variable region specifically binding CD3, and a heavy chain constant region,
ii) a second polypeptide chain, comprising, from N-terminus to C-terminus, a heavy chain variable region specifically binding BCMA, a first linker, a light chain variable region specifically binding BCMA, a second linker, a light chain variable region specifically binding CD3 and a light chain constant region; or alternatively a light chain variable region specifically binding BCMA, a first linker, a heavy chain variable region specifically binding BCMA, a second linker, a light chain variable region specifically binding CD3 and a light chain constant region,
iii) a third polypeptide chain, comprising, from N-terminus to C-terminus, a heavy chain variable region specifically binding GPRC5D, and a heavy chain constant region, and
iv) a fourth polypeptide chain, comprising, from N-terminus to C-terminus, a light chain variable region specifically binding GPRC5D, and a light chain constant region.

The antigen binding domain against BCMA may be an antibody or an antigen-binding portion thereof that specifically binds BCMA, wherein the heavy chain variable region thereof may comprise a VH-CDR1, a VH-CDR2 and a VH-CDR3 of SEQ ID NOs: 1-3, respectively, and the light chain variable region thereof may comprise a VL-CDR1, a VL-CDR2 and a VL-CDR3 of SEQ ID NOs: 4-6, respectively. The heavy chain variable region and the light chain variable region in the antigen binding domain against BCMA may comprise amino acid sequences having at least 85%, 86%, 87%, 88%, 89%, 90%, 91%, 92%, 93%, 94%, 95%, 96%, 97%, 98%, 99% or 100% sequence identity to SEQ ID NOs: 7 and 8, respectively.

The antigen binding domain against CD3 may be an antibody or an antigen-binding portion thereof that specifically binds CD3, wherein the heavy chain variable region thereof may comprise a VH-CDR1, a VH-CDR2 and a VH-CDR3 of SEQ ID NOs: 9-11, respectively, and the light chain variable region thereof may comprise a VL-CDR1, a VL-CDR2 and a VL-CDR3 of SEQ ID NOs: 12-14, respectively. The heavy chain variable region and the light chain variable region in the antigen binding domain against CD3 may comprise amino acid sequences having at least 85%, 86%, 87%, 88%, 89%, 90%, 91%, 92%, 93%, 94%, 95%, 96%, 97%, 98%, 99% or 100% sequence identity to SEQ ID NOs: 15 and 16, respectively.

The antigen binding domain against GPRC5D may be an antibody or an antigen-binding portion thereof that specifically binds GPRC5D, wherein the heavy chain variable region thereof may comprise a VH-CDR1, a VH-CDR2 and a VH-CDR3 of SEQ ID NOs: 17-19, respectively, and the light chain variable region thereof may comprise a VL-CDR1, a VL-CDR2 and a VL-CDR3 of SEQ ID NOs: 20-22, respectively. The heavy chain variable region and the light chain variable region in the antigen binding domain against GPRC5D may comprise amino acid sequences having at least 85%, 86%, 87%, 88%, 89%, 90%, 91%, 92%, 93%, 94%, 95%, 96%, 97%, 98%, 99% or 100% sequence identity to SEQ ID NOs: 23 and 24, respectively.

The heavy chain constant regions in the first polypeptide chain and the third polypeptide chain may be heavy chain constant regions with weak or no binding to FcR, preferably heavy chain constant regions with no binding to FcR, such as human IgG1 (N297A), human IgG1 (L234A+L235A), human IgG1 (L234A+L235A+P329G/A), human IgG1 (L234A+L235A+N297A), human IgG1 (L234A+L235A+N297A+P329G/A), human IgG2 (V234A+V237A), or human IgG1 (L234A+V235E) constant region, or human IgG4 constant region.

With respect to the heavy chain constant region in the first polypeptide chain and the heavy chain constant region in the third polypeptide chain, one may be a heavy chain constant region with a knob mutation, such as human IgG1 or IgG4 heavy chain constant region with the T366W mutation, or a functional fragment thereof. The other of the heavy chain constant region in the first polypeptide chain and the heavy chain constant region in the third polypeptide chain may be a heavy chain constant region with a hole mutation, such as human IgG1 or IgG4 heavy chain constant region with T366S/L368A/Y407V mutations, or a functional fragment thereof. In certain embodiments, one of the heavy chain constant region in the first polypeptide chain and the heavy chain constant region in the third polypeptide chain may be a heavy chain constant region with a knob mutation and weak or no binding to FcR, such as human IgG1 heavy chain constant region with L234A/L235A/N297A/T366W, e.g., set forth in SEQ ID NO: 25 (X1=W, X2=L, X3=Y). In certain embodiments, the other of the heavy chain constant region in the first polypeptide chain and the heavy chain constant region in the third polypeptide chain may be a heavy chain constant region with a hole mutation and weak or no binding to FcR, such as human IgG1 heavy chain constant region with L234A/L235A/N297A/T366S/L368A/Y407V, e.g., set forth in SEQ ID NO: 25 (X1=S, X2=A, X3=V).

The light chain constant region(s) in the second polypeptide chain and/or the fourth polypeptide chain may be e.g., human y light chain constant region. In certain embodiments, the light chain constant region comprises the amino acid sequence of SEQ ID NO: 26.

The first linker and the second linker may be a peptide of about 5-30 amino acids. In certain embodiments, the linker may be a peptide of 5-20 amino acids. In certain embodiments, the linker may be a GS linker, such as a GS linker comprising SEQ ID NOs: 36 or 37.

The first polypeptide chain, the second polypeptide chain, the third polypeptide chain and the fourth polypeptide chain in the multi-specific antibody of the disclosure, in certain embodiments, comprise amino acid sequences having at least 85%, 86%, 87%, 88%, 89%, 90%, 91%, 92%, 93%, 94%, 95%, 96%, 97%, 98%, 99% or 100% sequence identity to i) SEQ ID NOs: 27, 28, 29 and 30, respectively, or ii) SEQ ID NOs: 27, 16, 29 and 24, respectively.

The multi-specific antibody of the disclosure may i) specifically bind BCMA (human and monkey BCMAs), with slightly better binding ability than the prior art antibodies such as EM801, hardly affected by the soluble BCMAs in the environment, ii) bind CD3, with binding ability significantly lower than that of the prior art antibodies such as JNJ-64407564 (hereinafter abbreviated as JNJ) and EM801, activate the CD3 signaling only when the multi-specific antibody binds BCMA and/or GPRC5D to enable antibody "cross-linking", and show weaker T cell activation ability than JNJ and/or EM801 in the presence of certain tumor cells such as tumor cells highly expressing BCMA and GPRC5D, iii) bind GPRC5D with comparable or a bit higher binding ability as compared to JNJ, vi) kill tumor cells *in vitro,* with the killing ability higher than those of JNJ and EM801, v) have *in vivo* anti-tumor effect, with the effect evidently higher than that of EM801 and comparable to that of JNJ, iv) induce nearly no *in vivo* toxicity at the test concentrations.

Compared to the currently available bi-specific BCMA × CD3 antibodies and bi-specific GPRC5D × CD3 antibodies, the tri-specific antibody of the disclosure may more comprehensively kill BCMA⁺ tumor cells, GPRC5D⁺ tumor cells and BCMA⁺GPRC5D+ tumor cells, and better addressing the tumor antigen expression heterogeneity issue. That is, the tumor cells can be targeted and killed by the tri-specific antibody of the disclosure, no matter the BCMA and GPRC5D expression levels are high or low.

In addition, the ability of the multi-specific antibody of the disclosure to activate T cells is lower than those of JNJ and EM801 in the presence of tumor cells highly expressing BCMA and GPRC5D; the ability of the multi-specific antibody of the disclosure to activate T cells is lower than that of JNJ in the presence of tumor cells with high GPRC5D expression and low BCMA expression; the ability of the multi-specific antibody of the disclosure to activate T cells is lower than that of EM801 in the presence of tumor cells with high BCMA expression and low GPRC5D expression. In other words, the tri-specific antibody of the disclosure may more comprehensively kill tumor cells and produce lower toxic side effects, when addressing the tumor antigen expression heterogeneity problem.

The disclosure also provides a nucleic acid molecule encoding the multi-specific antibody of the disclosure, an expression vector that comprises the nucleic acid, and a host cell that comprises the expression vector or has the nucleic acid integrated in its genome. The disclosure further provides a method for preparing the multi-specific antibody using the host cell comprising the expression vector above, comprising (i) expressing the multi-specific antibody in the host cell and (ii) isolating the multi-specific antibody from the host cell or its cell culture.

The disclosure also provides a pharmaceutical composition, which comprises an effective amount of the multi-specific antibody, the nucleic acid molecule, the expression vector, or the host cell, of the disclosure, and a pharmaceutically acceptable carrier.

In a second aspect, the disclosure provides a method for treating or alleviating a tumor or cancer in a subject, comprising administering to the subject an effective amount of the pharmaceutical composition of the disclosure.

The tumor or cancer may be associated with BCMA and/or GPRC5D, including, but not limited to, multiple myeloma, and other malignant hematological tumors such as plasmocytoma, plasma cell leukemia, macroglobulinemia, solitary plasmacytoma of bone, and extramedullary plasmacytoma.

In certain embodiments, the tumor or cancer is multiple myeloma.

In certain embodiments, the tumor or cancer is multiple myeloma, and the myeloma cells highly express BCMA and GPRC5D.

In certain embodiments, the subject is a mammalian, particularly human.

The disclosure also provides the use of the multi-specific antibody, the nucleic acid molecule, the expression vector, the host cell or the pharmaceutical composition of the disclosure in preparation of a medicament for treating or alleviating a tumor or cancer.

All documents cited or referenced in the present application (including, but not limited to, all references, patents, published patent applications cited herein) ("herein cited documents"), and all documents cited or referenced in herein cited documents, together with any manufacturer's instructions, descriptions, product specifications, and product sheets for any products mentioned in the present application or any herein cited document, are hereby incorporated herein by reference, and may be employed in the practice of the invention. More specifically, all referenced documents are incorporated by reference to the same extent as if each individual document was specifically and individually indicated to be incorporated by reference. Any Genbank sequences mentioned in this disclosure are incorporated by reference.

### BRIEF DESCRIPTION OF THE DRAWINGS

The following detailed description, given by way of example, but not intended to limit the invention solely to the specific embodiments as described, may best be understood in conjunction with the accompanying drawings.
FIG. 1 is a schematic diagram of the structure of an exemplary tri-specific GPRC5D × BCMA × CD3 antibody.
FIG. 2 shows the binding activity of an exemplary tri-specific GPRC5D × BCMA × CD3 antibody (MBS314), a bi-specific BCMA × CD3 antibody (EM801), and a bi-specific GPRC5D × CD3 antibody (JNJ) to HEK293A/human BCMA cells (A), HEK293A/monkey BCMA cells (B), HEK293A/mouse BCMA cells (C), HEK293T/human GPRC5D cells (D), HEK293T/monkey GPRC5D cells (E), and human Jurkat T cells (F).
FIG. 3 shows the FACS analysis chart of the BCMA and GPRC5D expression on 3 multiple myeloma cell lines (A), and the exemplary tri-specific GPRC5D × BCMA × CD3 antibody's activity to activate CD3 signaling in Jurkat-NFAT-Luc cells after being "cross-linked" by these 3 cell lines (B).
FIG. 4 shows the GPRC5D and BCMA expression of MM.1S myeloma cells (A), and the growth curve of MM.1S tumors in mice treated by the exemplary tri-specific GPRC5D × BCMA × CD3 antibody (B).
FIG. 5 shows the FACS analysis chart of monocytes that were isolated from the fresh samples from relapsed/refractory multiple myeloma patients, treated by the exemplary tri-specific GPRC5D × BCMA × CD3 antibody and stained for CD138⁺ (A), and the statistic chart showing the percentage of CD138⁺ cells (B).
FIG. 6 shows the body weight change of C57BL/6 mice and CD3-humanized mice injected with the exemplary tri-specific GPRC5D × BCMA × CD3 antibody, wherein B6 represents C57BL/6 mice and CD3 represents CD3-humanized mice.
FIG. 7 is a line graph showing the PK of the exemplary tri-specific GPRC5D × BCMA × CD3 antibody in mice.

### DETAILED DESCRIPTION OF THE INVENTION

To ensure that the present disclosure may be more readily understood, certain terms are first defined. Additional definitions are set forth throughout the detailed description.

The term "CD3" refers to cluster of differentiation 3, comprising the γ chain, δ chain, ε chain and ζ chain. The term "CD3ε" or "CD3E" refers to CD3' ε chain. The term "CD3δ" or "CD3D" refers to CD3's δ chain. The term "CD3D&E" or "CD3δ&ε" refers to the εδ complex formed by the δ chain and the ε chain. These terms comprise the variants, homologs, orthologs and paralogs.

The term "BCMA" refers to B cell maturation antigen that is selectively expressed on malignant plasma cells in large amounts, and includes the variants, homologs, orthologs and paralogs. The term "human BCMA" refers to the BCMA protein having the amino acid sequence from human beings, such as the amino acid sequence of SEQ ID NO: 31. The term "monkey BCMA" refers to the BCMA protein having the amino acid sequence from monkeys, such as the amino acid sequence of SEQ ID NO: 32. The term "mouse BCMA" refers to the BCMA protein having the amino acid sequence from mice, such as the amino acid sequence of SEQ ID NO: 33.

The term "GPRC5D" refers to G protein-coupled receptor class C group 5 member D, a marker molecule of myeloma cells, and includes the variants, homologs, orthologs and paralogs. The term "human GPRC5D" refers to the GPRC5D protein having the amino acid sequence from human beings, such as the amino acid sequence of SEQ ID NO: 34. The term "monkey GPRC5D" refers to the GPRC5D protein having the amino acid sequence from monkeys, such as the amino acid sequence of SEQ ID NO: 35.

The term "optional" or "optionally" means the inclusion of some components or steps that are not compulsory or necessary, i.e., some components or steps are included in certain circumstances and not included in some other circumstances.

The term "antibody" as referred to herein includes IgG, IgA, IgD, IgE and IgM whole antibodies and any antigen binding fragment (i.e., "antigen-binding portion") thereof. A "whole antibody" or "full-length antibody" refers to a glycoprotein comprising at least two heavy (H) chains and two light (L) chains, wherein the heavy chain and the light chain are inter-connected by disulfide bonds; while a "half-antibody" is a half of a "whole antibody", comprising e.g., one heavy chain and one light chain, wherein the heavy chain and the light chain are inter-connected by disulfide bonds. Each heavy chain is comprised of a heavy chain variable region (abbreviated herein as V_{H}) and a heavy chain constant region. The heavy chain constant region is comprised of three domains, C_{H1}, C_{H2} and C_{H3}. Each light chain is comprised of a light chain variable region (abbreviated herein as V_{L}) and a light chain constant region. The light chain constant region is comprised of one domain, C_{L}. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies can mediate the binding of the immunoglobulin to host tissues or factors, including various cells of the immune system (e.g., effector cells) and the first component (Clq) of the classical complement system. The antibody constant region of the disclosure have been designed to have weak or no binding to the cells of the immune system and the protein of the complement system. It has been shown that the antigen-binding function of an antibody can be performed by fragments of a full-length antibody, including, but not limited to, (i) a Fab fragment, a monovalent fragment consisting of the V_{L} V_{H}, C_{L} and C_{H1} domains; (ii) a F(ab')₂ fragment, a bivalent fragment comprising two Fab fragments linked by a disulfide bridge at the hinge region; (iii) a Fd fragment consisting of the V_{H} and C_{H1} domains; (iv) a Fv fragment consisting of the V_{L} and V_{H} domains of a single arm of an antibody, (v) a dAb fragment (Ward et al., (1989) Nature 341:544-546), which consists of a V_{H} domain; (vi) an isolated complementarity determining region (CDR); and (viii) a nanobody, a heavy chain variable region containing a single variable domain and two constant domains. The "IgG-like antibody" herein refers to an antibody obtained by retaining the basic configuration of an IgG antibody and additionally adding some moieties such as antigen binding domains. A "functional fragment" of a heavy chain constant region herein refers to a fragment of the heavy chain constant region that retains the required functions (for example, to bind another heavy chain constant region or a fragment, to bind Fc receptors and/or complement system component).

The term "agonistic anti-CD3 antibody" used herein refers to an anti-CD3 antibody that can bind CD3 and activate or trigger CD3 signaling to promote activation and proliferation of immune cells such as T cells. The anti-CD3 antibodies of the disclosure activate CD3 signaling and induce immune cell activity only when they are "cross-linked".

The term "cross-link" or "cross-linking" refers to the antibody aggregation or interaction induced by binding the BCMA targeted portion in the multi-specific antibody to BCMA, binding the GPRC5D targeted portion to GPRC5D, and/or binding the Fc portion to the Fc receptor and/or the complement system component (in case of Fc portion that possesses binding ability to FcR and/or complement system component). In *in vitro* tests, antibody cross-linking may occur when the antibody Fc portion binds to an anti-Fc secondary antibody. In contrast, the term "free" means the antibodies do not interact with each other or with other molecules that may cause dimerization or multimerization. The multi-specific antibody of the disclosure, when in the "free" status, does not activate CD3 signaling or activate immune cells such as T cells.

The term "antagonistic anti-BCMA antibody" or "blocking anti-BCMA antibody" refers to an antibody that can bind BCMA and block or inhibit BCMA signaling mediated by the interaction of BCMA with its ligand such as BAFF or APRIL, especially APRIL. The antagonistic anti-BCMA antibody may block proliferation and apoptosis evasion of malignant tumor cells. The antigen binding domain against BCMA in the multi-specific antibody of the disclosure may be antagonistic, binding to BCMA and blocking BCMA-APRIL binding/interaction without inducing BCMA signaling.

The term "FcR" refers to a protein expressed on the surface of certain cells such as B lymphocytes, natural killer cells, and macrophages, which can be bound by the Fc portion of an antibody, stimulating phagocytosis of and toxicity to target cells and thus playing key roles in immune system. The FcR includes, Fcα receptor, Fcε receptor and Fcy receptor, wherein the Fcy receptor belongs to the immunoglobulin superfamily and is the most important FcR for inducing phagocytosis of microbes, including FcγRI (CD64), FcγRIIA (CD32A), FcγRIIB (CD32B), and FcγRIIIA (CD16A).

The term "multi-specific" antibody refers to an antibody that specifically binds two or more (e.g., three) target molecules, or two or more (e.g., three) different epitopes in a same target molecule. The multi-specific molecule includes the antibody of the disclosure that specifically binds BCMA, GPRC5D and CD3. A "bi-specific antibody" refers to, in certain circumstances, an antibody that specifically binds two target cells or two epitopes on a target molecule. In contrast, a "monospecific" antibody refers to an antibody that specifically binds a certain target molecule, especially an epitope in a certain target molecule.

As used herein, an antibody that "specifically binds BCMA", "specifically binds CD3" or "specifically binds GPRC5D" refers to an antibody that binds BCMA, CD3 or GPRC5D but does not substantially bind to non-BCMA, non-CD3 or non-GPRC5D proteins. Preferably, the antibody binds the human BCMA, CD3 or GPRC5D protein with "high affinity", i.e., with a K_{D} at 5.0 × 10⁻⁷ M or lower.

The term "does not substantially bind" to a protein or cell, means does not bind or does not bind with a high affinity to a protein or cell, i.e. binds to a protein or cell with a K_{D} of 1.0 × 10⁻⁶ M or more.

The term "EC₅₀", also known as half maximal effective concentration, refers to the concentration of an antibody which produces 50% of its maximum effect.

The term "IC₅₀", also known as half maximal inhibitory concentration, refers to the concentration of a drug or an inhibitor which inhibits a specific biological process by 50%.

The term "subject" includes any human or nonhuman animal. The term "nonhuman animal" includes all vertebrates, e.g., mammals and non-mammals, such as non-human primates, sheep, dogs, cats, cows, horses, chickens, amphibians, and reptiles, although mammals are preferred, such as non-human primates, sheep, dogs, cats, cows and horses.

The term "effective amount" refers to an amount of the antibody of the present disclosure sufficient to achieve the expected results. The term "therapeutically effective amount" refers to an amount of the antibody of the disclosure sufficient to prevent or ameliorate the symptoms associated with a disease or condition (such as a cancer). A therapeutically effective amount is related to the disease to be treated, where the actual effective amount can be readily discerned by those of skill in the art.

### Advantageous properties of multi-specific antibodies of the disclosure

The multi-specific antibodies of the disclosure, when compared to the prior art antibodies such as JNJ and EM801, have i) comparable or higher BCMA binding ability, ii) comparable or higher GPRC5D binding ability, iii) significantly lower CD3 binding ability, vi) comparable or better *in vitro* anti-tumor activity, v) comparable or better *in vivo* anti-tumor efficacy, and/or iv) comparable or lower *in vivo* toxicity.

The inventors of the application, constructed tri-specific GPRC5D × BCMA × CD3 antibodies with elegant design that possess potent tumor killing ability and induce relatively low toxicity, surmounting and ameliorating the efficacy-toxicity-balance problem of the bi-specific anti-CD3 antibodies currently used in MM treatment, including the BCMA × CD3 bi-specific antibodies and the GPRC5D × CD3 bi-specific antibodies. Compared to the currently available BCMA × CD3 bi-specific antibodies and GPRC5D × CD3 bi-specific antibodies, the tri-specific antibodies of the disclosure may more comprehensively kill BCMA⁺ tumor cells, GPRC5D⁺ tumor cells, and BCMA⁺GPRC5D⁺ tumor cells, and better addressing the tumor antigen expression heterogeneity issue. That is, the tumor cells can be targeted and killed by the tri-specific antibodies of the disclosure, no matter the BCMA and GPRC5D expression levels on these cells are high or low.

In addition, the ability of the multi-specific antibodies of the disclosure to activate T cells is lower than those of JNJ and EM801 in the presence of tumor cells highly expressing BCMA and GPRC5D; the ability of the multi-specific antibodies of the disclosure to activate T cells is lower than that of JNJ in the presence of tumor cells with high GPRC5D expression and low BCMA expression; the ability of the multi-specific antibodies of the disclosure to activate T cells is lower than that of EM801 in the presence of tumor cells with high BCMA expression and low GPRC5D expression. In other words, the tri-specific antibodies of the disclosure may more comprehensively kill tumor cells and produce lower toxic side effects, when addressing the tumor antigen expression heterogeneity problem. In particular, the multi-specific antibodies of the disclosure have better killing effect and produce relatively low toxic side effects, against tumors highly expressing both BCMA and GPRC5D.

The heavy chain variable region CDRs and light chain variable region CDRs of the monospecific antibodies or antigen binding fragments thereof used in the multi-specific antibodies of the disclosure have been defined by the Kabat numbering system. As is well known in the art, the heavy chain variable region and light chain variable region CDRs can also be determined by other systems such as Chothia, IMGT, AbM, or Contact numbering system/method.

The multi-specific antibodies of the disclosure includes bi-specific antibodies.

### Multi-specific antibodies of the disclosure

A multi-specific antibody of the disclosure may comprise: i) an antigen binding domain against CD3, ii) an antigen binding domain against BCMA, and iii) an antigen binding domain against GPRC5D. The antigen binding domain against CD3 may be an agonistic antigen binding domain, which may specifically bind CD3 and activate CD3 signaling. The antigen binding domain against BCMA may bind BCMA and optionally block BCMA-APRIL binding/interaction, i.e., may optionally be an antagonistic antigen binding domain. The antigen binding domain against GPRC5D may specifically bind GPRC5D.

A multi-specific antibody of the disclosure may comprise e.g., one antigen binding domain against CD3, one antigen binding domain against BCMA, and one antigen binding domain against GPRC5D.

A multi-specific antibody of the disclosure may be an IgG-like antibody, comprising an antigen binding domain against CD3 in the Fab or Fv format, an antigen binding domain against GPRC5D in the Fab or Fv format, and an antigen binding domain against BCMA in the single chain fragment variable (scFv) format.

The multi-specific antibody may comprise i) an anti-CD3 half-antibody, comprising a heavy chain variable region, a heavy chain constant region, a light chain variable region, and an optional light chain constant region, ii) an anti-GPRC5D half-antibody, comprising a heavy chain variable region, a heavy chain constant region, a light chain variable region, and an optional light chain constant region, and iii) an antigen binding domain against BCMA in the scFv format, comprising a heavy chain variable region, an optional first linker, and a light chain variable region, wherein the anti-CD3 half-antibody and the anti-GPRC5D half-antibody may form an IgG full-length antibody, the antigen binding domain against BCMA in the scFv format may be linked, optionally via a second linker, to N-terminus of the heavy chain variable region, N-terminus of the light chain variable region, C-terminus of the heavy chain constant region or C-terminus of the light chain constant region of the anti-CD3 half-antibody. In certain embodiments, the antigen binding domain against BCMA in the scFv format may be linked to N-terminus of the heavy chain variable region or N-terminus of the light chain variable region of the anti-CD3 half-antibody.

The antigen binding domain against BCMA in the scFv format may comprise, from N-terminus to C-terminus, a heavy chain variable region, an optional first linker, and a light chain variable region; or a light chain variable region, an optional first linker, and a heavy chain variable region.

One heavy chain constant region in the two half-antibodies, may be a heavy chain constant region with a knob mutation and weak or no binding to FcR, such as human IgG1 heavy chain constant region with L234A/L235A/N297A/T366W, and the other heavy chain constant region may be a heavy chain constant region with a hole mutation and weak or no binding to FcR, such as human IgG1 heavy chain constant region with L234A/L235A/N297A/T366S/L368A/Y407V.

The first linker and the second linker may be made up of amino acids linked together by peptide bonds, preferably from 5 to 30 amino acids linked by peptide bonds, wherein the amino acids are selected from the 20 naturally occurring amino acids. One or more of these amino acids may be glycosylated, as is understood by those of skill in the art. In one embodiment, the 5 to 30 amino acids may be selected from glycine, alanine, proline, asparagine, glutamine, serine and lysine. In one embodiment, the linker is made up of a majority of amino acids that are sterically unhindered, such as glycine and alanine. Exemplary linkers are polyglycines, particularly poly(Gly-Ala), and polyalanines. The exemplary linker of the disclosure may comprise the amino acid sequence of SEQ ID NOs: 19, 20, 21 or 22.

The linker may also be a non-peptide linker. For example, alkyl linkers such as -NH-, - (CH₂)s-C(O)-, wherein s=2-20, can be used. These alkyl linkers may further be substituted by any non-sterically hindering group such as lower alkyl (e.g., C₁₋₆ lower acyl), halogen (e.g., CI, Br), CN, NH₂, phenyl, etc.

### Conservative modifications

In another embodiment, the multi-specific antibody of the disclosure may comprise a heavy chain variable region and/or a light chain variable region or the CDR1, CDR2 and CDR3 sequences with one or more conservative modifications. It is understood in the art that certain conservative sequence modifications do not remove antigen binding capability.

As used herein, the term "conservative sequence modification" is intended to refer to amino acid modifications that do not significantly affect or alter the binding characteristics of the antibody. Such conservative modifications include amino acid substitutions, additions and deletions. Modifications can be introduced into an antibody of the disclosure by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within the CDR regions of an antibody of the disclosure can be replaced with other amino acid residues from the same side chain family and the obtained antibody can be tested for the retained function(s) (i.e., the function(s) set forth above) using the functional assays described herein.

### Genetically engineered antibodies

The multi-specific antibodies of the disclosure can be prepared using an antibody having one or more of the V_{H}/V_{L} sequences of the present disclosure, as starting material to engineer a modified antibody. An antibody can be engineered by modifying one or more residues within one or both variable regions (i.e., V_{H} and/or V_{L}), for example within one or more CDR regions and/or within one or more framework regions, to improve the binding affinity and/or increase the similarity to the antibodies naturally occurred in certain species. For example, the framework regions are modified to provide humanized antibodies. Additionally or alternatively, an antibody can be engineered by modifying residues within the constant region(s), for example to alter the effector function(s) of the antibody.

Therefore, each heavy chain variable region and/or each light chain variable region contained in the multi-specific antibodies of the disclosure may contain the VH-CDR1, VH-CDR2, and VH-CDR3, and/or the VL-CDR1, VL-CDR2 and VL-CDR3 of the disclosure, but different framework sequences.

The inventors of the application found that, when an antigen binding domain is in the scFv format, the stability may be inferior to that of its full-length antibody or Fab format. Therefore, in one embodiment of the disclosure, to solve the stability problem that may be caused by the antigen binding domain against BCMA in the scFv format when used in the construction of the multi-specific antibody, the framework sequences in the anti-BCMA heavy/light chain variable region(s), such as the amino acids in the light chain variable region FR3 and FR4 portions were modified based on computer modeling, to enhance scFv's stability, reducing the formation of aggregates and increasing monomer levels. The Fab format and the scFv format, with or without the modification(s), showed comparable binding affinity, BCMA-APRIL blocking activity and anti-tumor efficacy.

The inventors of the application further found that, the multi-specific antibodies may be further improved in druggability and therefore the industrial production levels by changing the framework sequences in the anti-BCMA heavy/light chain variable region(s), e.g., changing the hydrophilicity/hydrophobicity of the amino acids in the framework region(s).

Engineered antibodies of the disclosure include those in which modifications have been made to framework residues within V_{H} and/or V_{L}, to change the antibodies' characteristic. Typically, the framework regions are modified to reduce the antibody's immunogenicity. For example, one approach is to "back-mutate" one or more framework residues to the corresponding germline sequence(s). More specifically, an antibody that has undergone somatic mutation can contain framework residues that differ from the germline sequence from which the antibody is derived. Such residues can be identified by comparing the antibody framework sequences to the germline sequences from which the antibody is derived.

Another type of framework modification involves mutating one or more residues within the framework region, or even within one or more CDR regions, to remove T cell epitopes to thereby reduce the potential immunogenicity of the antibody. This approach is also referred to as "deimmunization" and is described in further detail in U.S. Patent Publication No. 20030153043.

In addition, or as an alternative to modifications made within the framework or CDR regions, the antibodies of the disclosure can be engineered to include modifications within the Fc region, typically to alter one or more functional properties of the antibodies, such as serum half-life, complement fixation, Fc receptor binding, and/or antigen-dependent cellular cytotoxicity. Furthermore, the antibodies of the disclosure can be chemically modified (e.g., by attaching one or more chemical moieties to the antibodies) or be modified to alter the glycosylation, again to alter one or more functional properties of the antibodies.

In one embodiment, the hinge region of C_{H1} is modified such that the number of cysteine residues in the hinge region is altered, e.g., increased or decreased. This approach is described further in U.S. Pat. No. 5,677,425. The number of cysteine residues in the hinge region of C_{H1} is altered to, for example, facilitate assembly of the light and heavy chains or to increase or decrease the antibody stability.

In another embodiment, the Fc hinge region of the antibodies is mutated to decrease the biological half-life of the antibodies. More specifically, one or more amino acid mutations are introduced into the C_{H2}-C_{H3} domain interface region of the Fc-hinge fragment such that the antibodies have impaired Staphylococcyl protein A (SpA) binding relative to native Fc-hinge domain SpA binding. This approach is described in further detail in U.S. Pat. No. 6,165,745.

In still another embodiment, the glycosylation of the antibodies is modified. For example, a de-glycosylated antibody can be made (i.e., the antibody lacks glycosylation). Glycosylation can be altered to, for example, increase the affinity of the antibody for the antigen. Such carbohydrate modifications can be accomplished by, for example, altering one or more sites of glycosylation in the antibody sequences. For example, one or more amino acid substitutions can be made that result in elimination of one or more variable region framework glycosylation sites to thereby eliminate glycosylation at that site. Such de-glycosylation may increase the affinity of the antibody for the antigen. See, e.g., U.S. Pat.Nos. 5,714,350 and 6,350,861. Additionally, an antibody can be made that has an altered type of glycosylation, such as a hypo-fucosylated antibody having reduced amounts of fucosyl residues, or an antibody having increased bisecting GlcNac structures. Such altered glycosylation patterns have been demonstrated to increase the ADCC ability of the antibody. Such carbohydrate modifications can be accomplished by, for example, expressing the antibody in a host cell with altered glycosylation machinery. Cells with altered glycosylation machinery have been described in the art and can be used as host cells for expressing the recombinant antibodies of the disclosure to thereby produce antibodies with altered glycosylation. For example, the cell lines Ms704, Ms705, and Ms709 lack the fucosyltransferase gene, FUT8 (α (1, 6)-fucosyltransferase), such that antibodies expressed in the Ms704, Ms705, and Ms709 cell lines lack fucose on their carbohydrates.

Another modification of the antibodies herein is pegylation (PEGylation). An antibody can be pegylated to, for example, increase the antibody's biological (e.g., serum) half-life. To pegylate an antibody, the antibody or the fragment thereof typically is reacted with polyethylene glycol (PEG), such as a reactive ester or aldehyde derivative of PEG, under conditions in which one or more PEG groups become attached to the antibody or antibody fragment.

### Nucleic acid molecules encoding antibodies of the disclosure

In another aspect, the disclosure provides a nucleic acid molecule that encodes e.g., the anti-BCMA heavy chain variable region-first linker-anti-BCMA light chain variable region-second linker-anti-CD3 heavy chain variable region-heavy chain constant region, the anti-GPRC5D heavy chain variable region-heavy chain constant region, anti-GPRC5D light chain variable region-light chain constant region, of the multi-specific antibodies of the disclosure.

The nucleic acid can be present in whole cells, in a cell lysate, or in a partially purified or substantially pure form. A nucleic acid is "isolated" or "substantially pure" when purified away from other cellular components or other contaminants, e.g., other cellular nucleic acids or proteins, by standard techniques. A nucleic acid of the disclosure can be, e.g., DNA or RNA and may or may not contain intronic sequences. In a preferred embodiment, the nucleic acid is a cDNA molecule.

The nucleic acid molecule of the disclosure can be obtained using standard molecular biology techniques. Preferred nucleic acids molecules of the disclosure include those encoding the V_{H} and V_{L} sequences or CDRs of the anti-CD3, anti-BCMA, and anti-GPRC5D monospecific antibodies. Once DNA fragments encoding V_{H} and/or V_{L} segments are obtained, these DNA fragments can be further manipulated by standard recombinant DNA techniques, for example to convert the variable region genes to full-length antibody chain genes, to Fab fragment genes or to a scFv gene. In these manipulations, a V_{L}- or V_{H}-encoding DNA fragment is operatively linked to another DNA fragment encoding another protein, such as an antibody constant region or a flexible linker. The term "operatively linked" means that two DNA fragments are joined such that the amino acid sequences encoded by the two DNA fragments remain in-frame.

To create a scFv gene, the V_{H}- and V_{L}-encoding DNA fragments are operatively linked to another fragment encoding a flexible linker, e.g., encoding the amino acid sequence (Gly₄-Ser)₃, such that the V_{H} and V_{L} sequences can be expressed as a contiguous single-chain protein, with the V_{L} and V_{H} regions joined by the flexible linker (see, e.g., Bird et al., (1988) Science 242:423-426; Huston et al., (1988) Proc. Natl. Acad. Sci. USA 85:5879-5883; McCafferty et al., (1990) Nature 348:552-554).

For the multi-specific antibodies of the disclosure, sequences encoding the CDRs, VHs and VLs of the anti-CD3, anti-BCMA and anti-GPRC5D antibodies are firstly obtained, and then combined according to the required structures of the multi-specific antibodies. For example, the sequences coding for the anti-BCMA heavy chain variable region, the first linker, the anti-BCMA light chain variable region, the second linker, the anti-CD3 heavy chain variable region and the heavy chain constant region may be operatively linked as required.

### Generation of antibodies of the disclosure

The multi-specific antibody of the disclosure may be produced by i) inserting the sequences encoding the polypeptide chains of the multi-specific antibody into one or more expression vectors which are operatively linked to regulatory sequences that control transcription or translation; (ii) transducing or transfecting host cells with the expression vectors; and (iii) expressing the polypeptide chains to form the multi-specific antibody of the disclosure.

The term "regulatory sequence" is intended to include promoters, enhancers and other expression control elements (e.g., polyadenylation signals) that control the transcription or translation of the antibody genes.

The expression vector can encode a signal peptide that facilitates secretion of the polypeptide chain(s) from a host cell. The antibody chain gene can be cloned into the vector such that the signal peptide is linked in-frame to the amino terminus of the antibody chain gene. The signal peptide can be an immunoglobulin signal peptide or a heterologous signal peptide (i.e., a signal peptide from a non-immunoglobulin protein).

In addition to the antibody polypeptide chain genes and regulatory sequences, the expression vectors of the disclosure can carry additional sequences, such as sequences that regulate replication of the vector in host cells (e.g., origins of replication) and selectable marker genes. For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr-host cells with methotrexate selection/amplification) and the neo gene (for G418 selection).

The expression vector(s) encoding different polypeptide chains of the multi-specific antibody can be transfected into a host cell by standard techniques. The various forms of the term "transfection" encompass a wide variety of techniques commonly used for the introduction of exogenous DNA into a prokaryotic or eukaryotic host cell, e.g., electroporation, calcium-phosphate precipitation, DEAE-dextran transfection and the like. Although it is theoretically possible to express the antibody of the disclosure in either prokaryotic or eukaryotic host cells, expression of the antibody in eukaryotic cells is preferred, and most preferably in mammalian host cells, because such eukaryotic cells, and in particular mammalian cells, are more likely than prokaryotic cells to assemble and secrete a properly folded and immunologically active antibody.

The expression vectors that can be used in the present application include, but are not limited to, plasmids, viral vectors, yeast artificial chromosomes (YACs), bacterial artificial chromosomes (BACs), transformation-competent artificial chromosomes (TACs), mammalian artificial chromosomes (MACs) and human artificial episomal chromosomes (HAECs).

### Pharmaceutical compositions

In another aspect, the present disclosure provides a pharmaceutical composition which may comprise one or more multi-specific antibodies, the multi-specific antibody-encoding nucleic acid molecules, the nucleic acid molecule-containing expression vectors, and/or the nucleic acid molecule-containing host cells, of the disclosure, formulated together with a pharmaceutically acceptable carrier. The pharmaceutical composition may optionally contain one or more additional pharmaceutically active ingredients, such as another anti-tumor drug, or an immune enhancement drug. The pharmaceutical composition of the disclosure may be used in combination with e.g., an additional anti-tumor agent, or another immune enhancement drug.

The pharmaceutical composition may comprise any number of excipients. Excipients that can be used include carriers, surfactants, thickening or emulsifying agents, solid binders, dispersion or suspension aids, solubilizers, colorants, flavoring agents, coatings, disintegrating agents, lubricants, sweeteners, preservatives, isotonic agents, and combinations thereof. The selection and use of suitable excipients are taught in Gennaro, ed., Remington: The Science and Practice of Pharmacy, 20th Ed. (Lippincott Williams & Wilkins 2003).

The pharmaceutical composition is suitable for oral, intravenous, intramuscular, subcutaneous, parenteral, spinal or epidermal administration (e.g., by injection or infusion). Depending on the route of administration, the active ingredient can be coated in a material to protect it from the action of acids and other natural conditions that may inactivate it. The phrase "parenteral administration" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion. Alternatively, an antibody of the disclosure can be administered via a non-parenteral route, such as a topical, epidermal or mucosal route of administration, e.g., intranasally, orally, vaginally, rectally, sublingually or topically. Preferably, the pharmaceutical composition is orally administered.

Pharmaceutical compositions can be in the form of sterile aqueous solutions or dispersions. They can also be formulated in a micro-emulsion, liposome, or other ordered structure suitable to high drug concentration.

The administration of the pharmaceutical composition of the disclosure may be determined by physicians, e.g., doctors, depending on the specific circumstances of a subject, e.g., sex, age, medical history and the like.

A "therapeutically effective amount" of the multi-specific antibody of the disclosure, may result in a decrease in severity of disease symptoms, or an increase in frequency and duration of disease symptom-free periods. For example, for the tumor-bearing subjects, a "therapeutically effective amount" preferably reduces tumor size by at least about 20%, more preferably by at least about 40%, even more preferably by at least about 60%, and still more preferably by at least about 80%, or even eliminate tumors, relative to control subjects.

The pharmaceutical composition can be a controlled release formulation, including implants, and microencapsulated delivery systems. Biodegradable, biocompatible polymers can be used, such as ethylene vinyl acetate, polyanhydrides, polyglycolic acid, collagen, polyorthoesters, and polylactic acid.

In certain embodiments, the antibodies of the disclosure can be formulated to ensure proper distribution *in vivo.* For example, to ensure that the therapeutic antibodies of the disclosure cross the blood-brain barrier, they can be formulated in liposomes, which may additionally comprise targeting moieties to enhance selective transport to specific cells or organs.

### Uses and methods of the disclosure

The pharmaceutical composition of the disclosure has multiple *in vitro* and *in vivo* applications. For example, it may be used to treat or alleviate tumors or cancers.

The pharmaceutical composition of the disclosure may be used to treat or alleviate BCMA and/or GPRC5D associated tumors or cancers, including, but not limited to, multiple myeloma, and other malignant hematological tumors such as plasmocytoma, plasma cell leukemia, macroglobulinemia, solitary plasmacytoma of bone, and extramedullary plasmacytoma.

The disclosure provides a combination therapy in which the pharmaceutical composition of the present disclosure is co-administered with one or more additional antibodies or non-antibody agents, such as an immune enhancement agent.

The combination of therapeutic agents discussed herein can be administered concurrently as a single composition in a pharmaceutically acceptable carrier, or concurrently as separate compositions with each agent in a pharmaceutically acceptable carrier. In another embodiment, the combination of therapeutic agents can be administered sequentially.

Furthermore, if more than one dose of the combination therapy is administered sequentially, the order of the sequential administration can be reversed or kept in the same order at each time point of administration, sequential administrations can be combined with concurrent administrations, or any combination thereof.

Various aspects and embodiments of the disclosure will be discussed in reference to the drawings and the following Examples. Other aspects and embodiments are apparent to those skilled in the art. All references described herein are incorporated herein by reference in their entirety. Although the present application has been described in reference to the exemplary embodiments, many equivalent changes and alterations, in view of the disclosure, are apparent to those skilled in the art. Therefore, the exemplary embodiments of the disclosure are only provided for illustration, and are not limiting. Various alterations may be made to the embodiments without departing from the sprit and scope of the application.

### Examples

### Example 1. Construction of cell lines stably expressing BCMA or GPRC5D

Cell lines stably expressing human BCMA, monkey BCMA, and mouse BCMA were respectively constructed using HEK293A cells. Briefly, cDNA sequences encoding human BCMA, monkey BCMA and mouse BCMA (amino acid sequences set forth in SEQ ID NOs: 31, 32 and 33, respectively) were synthesized, digested by enzymes, and then cloned into pLV-EGFP(2A)-Puro vectors (Beijing Inovogen, CN) between BamH1 and EcoR1. Lentiviruses were generated in HEK293T cells (Nanjing Cobioer, CN) by lipofection of the resultant pLV-EGFP(2A)-Puro-human BCMA, pLV-EGFP(2A)-Puro-monkey BCMA or pLV-EGFP(2A)-Puro-mouse BCMA with psPAX and pMD2.G plasmids, according to the instruction of Lipofectamine 3000 kit (Thermo Fisher Scientific, USA). Three days post transfection, the lentiviruses were harvested from the cell culture medium of HEK293T cells (DMEM medium (Cat#:SH30022.01, Gibco) supplemented with 10% FBS (Cat#:FND500, Excell). The lentiviruses were then used to infect HEK293A cells (Nanjing Cobioer, CN), so as to generate cells stably expressing human, monkey or mouse BCMA (referred to as HEK293A/human BCMA cells, HEK293A/monkey BCMA cells, HEK293A/mouse BCMA cells, respectively). These HEK293A cells were cultured in DMEM containing 10% FBS and 0.2 µg/ml puromycin (Cat#:A11138-03, Gibco) for 7 days. The expression of human and monkey BCMA was analyzed by FACS in a flow cytometer using a commercially available anti-BCMA antibody (PE-anti-human BCMA antibody, Cat#: 357503, Biolegend, USA). Similarly, the expression of mouse BCMA was confirmed by FACS using a commercially available anti-mouse BCMA antibody (anti-mouse BCMA antibody, Cat#: O88472, Novus, USA).

Similarly, HEK293T cell lines stably expressing human or monkey GPRC5D were constructed using cDNAs encoding human or monkey GPRC5D (amino acid sequences set forth in SEQ ID NOs: 34 and 35, respectively), and the expression of human and monkey GPRC5D was tested using an anti-GPRC5D antibody (the anti-GPRC5D antibody was prepared using the amino acid sequence 10 and 11 of the published patent application WO2022058445A1, at the final concentration of 20 µg/ml).

### Example 2. Construction and expression of tri-specific CD3×BCMA×GPRC5D antibodies

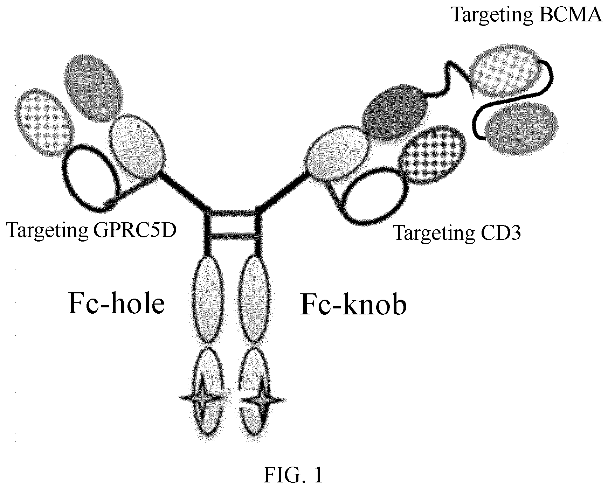

Tri-specific antibodies were constructed as asymmetrical whole antibodies with anti-BCMA: anti-CD3: anti-GPRC5D ratio at 1:1:1, the structure of an exemplary tri-specific antibody was shown in FIG. 1. Using the GS vector (see details in ZL200510064335.0) as the expression vector, two half-antibodies, MBS-314-knob and MBS-314-hole, of the exemplary tri-specific antibody were constructed. The MBS-314-knob's long chain contained anti-BCMA scFv-linker-anti-CD3 heavy chain variable region-heavy chain constant region, whose amino acid sequence was set forth in SEQ ID NO: 27, and its short chain contained anti-CD3 light chain variable region-light chain constant region, whose amino acid sequence was set forth in SEQ ID NO: 28. MBS-314-hole's long chain contained anti-GPRC5D heavy chain variable region-heavy chain constant region, whose amino acid sequence was set forth in SEQ ID NO: 29, and its short chain contained anti-GPRC5D light chain variable region-light chain constant region, whose amino acid sequence was set forth in SEQ ID NO: 30. In the BCMA targeted scFv, the framework (e.g., the fourth framework) of the light chain variable region was modified to stabilize the scFv structure.

DNA sequences encoding the anti-BCMA scFv-linker-anti-CD3 heavy chain variable region in the MBS-314-knob half antibody, and the anti-GPRC5D heavy chain variable region in the MBS-314-hole half-antibody were synthesized respectively. These two fragments were digested with restriction endonucleases *Eco*RI and *Nhe*I*,* and the resultant DNA fragments were cloned into the vectors containing the respective heavy chain constant region (amino acid sequence set forth in SEQ ID NO: 25 (X1=W, X2=L, X3=Y), SEQ ID NO: 25 (X1=S, X2=A, X3=V)), accomplishing the assembly of expression vectors with the 2 half-antibodies' long chain full-length sequences. In addition, DNA sequences encoding the anti-CD3 light chain variable region in the MBS-314-knob half antibody, and the anti-GPRC5D light chain variable region in the MBS-314-hole half-antibody were synthesized respectively. These two fragments were digested with restriction endonucleases *Cla*I and *BsiWI,* and the resultant DNA fragments were cloned into the vectors containing the light chain constant region (SEQ ID NO: 26), accomplishing the assembly of expression vectors with the 2 half-antibodies' short chain full-length sequences. The half-antibodies' short chain full-length sequences were digested with *Cla*I and *Hind*III, the half-antibodies' long chain full-length sequences were digested with *Eco*RI and *Xho*I*,* the pCMV-cofragment plasmid was digested with *Hind*III and *Eco*RI, and the GS-vector was digested with *Cla*I and *Xho*I. The four DNA fragments were harvested, ligated, and subjected to transformation, and monoclonal clones were picked up for sequencing, to obtain half-antibody expression vectors with correct sequences, which were named as MBS-314-knob expression vector and MBS-314-hole expression vector. The plasmids were extracted using TianGen Endo-Free Maxi Plasmid Extraction Kit (Cat#:DP117, TianGen). The plasmids were re-suspended in 5 ml FreeStyle F17 cell culture medium, and the transfection agent (PEI) at the volume 3 times that of the plasmids was re-suspended in 5 ml FreeStyle F17 cell culture medium. The PEI was slowly added to the plasmids, sufficiently mixed, and placed at room temperature for 15 min. The mixture obtained above was added to 100 ml 293F cells (cell density at 1 × 10⁶/ml). The HEK-293F cells, post transfection, were cultured in a 37°C and 5%CO₂ incubator at rotational speed of 120 RPM. After 10-12 days, the cell culture supernatants were harvested, centrifuged at 3500 rpm for 5 min, and flowed through a 0.22 µm film filter to remove the cell debris. The half-antibodies were enriched and purified using pre-equilibrated Protein-A affinity columns (Cat#: 17040501, GE, USA), and eluted with the elution buffer (20 mM citric acid, pH 3.0-3.5). The antibodies were stored in PBS (pH 7.0) and the antibody concentrations were determined using NanoDrop. The purified half-antibodies were subject to further assembly.

### Example 3. Assembly of tri-specific CD3xBCMAxGPRC5D antibody

The purified half-antibodies were assembled *in vitro.* Specifically, the MBS-314-knob half-antibody and the MBS-314-hole half-antibody were mixed at 1:1 molar ratio, added with Tris base buffer till pH 8.0 followed by some amount of reducing glutathione solution, and allowed to react overnight at 25°C with low-speed stirring. After the reaction was completed, the mixture was added with 2 M acetic acid solution to adjust pH to 5.5. The reducing agent was removed by ultrafiltration, to terminate the reaction. The antibodies as assembled were transferred to low-salt Tris buffer (pH8.0) and filtered using a 0.2 µm film filter. Firstly, anion exchange columns were balanced with low-salt Tris buffer (pH8.0), and loaded with the sample. The components that had passed through the columns were collected, and rinsed by low-salt Tris buffer (pH8.0) until UV280 trended to the baseline. The collected samples were adjusted to pH5.5 using an acetic acid solution, concentrated to 1 ml using a 30 kDa ultrafilter tube, and filtered using a 0.2 µm film filter. Then, cation exchange columns were balanced with a low-concentration acetate buffer (pH5.5), and loaded with the samples. After that, low-concentration acetate buffer (pH5.5) was used to balance the columns, and linear gradient elution was done using 20 CV 0-100% high concentration acetate solutions, and the components as eluted were collected.

The tri-specific antibody as assembled was designated as MBS314. The purified antibody with a purity higher than 90% as measured by mass spectrum, were further characterized below.

### Example 4. Binding affinity of CD3×BCMA×GPRC5D to human CD3, human GPRC5D and human BCMA

The binding affinity of the antibody MBS314 to human CD3, human GPRC5D and human BCMA were measured by the capture approach (BIAcore 8K). The mouse anti-human Fc antibody (Cat#:BR100839, cytiva) was coupled to the surface of a CM5 chip. The antibody to be tested was diluted to 1 µg/ml with the HBS-EP buffer (Cat#:BR-1006-69, GE Life Sciences), to ensure about 100 RU of the antibody was captured by the anti-human Fc antibody. The human BCMA antigen (Cat#:BCA-H522y, ACRO), human CD3D&E protein (Cat#: CT038-H2508H, Sino biological), and human GPRC5D antigen (Cat#:HM05P, Kactus Biosystems) were diluted with HBS-EP buffer to 0.5, 0.25, 0.125, 0.0625, 0.03125, 0.015625, 0.0078125, and 0.003900625 µg/ml, respectively. Each protein at different concentrations was flowed through the surface of the stationary phase. Chips were regenerated by 3 M MgCl₂ solution. The kinetics assay was run with the Kinetics Wizard in BIAcore 8K's control software. JNJ (a bi-specific GPRC5D×CD3 antibody) and EM801 (a bi-specific BCMA×CD3 antibody) were used as the positive controls, wherein JNJ was prepared according to the amino acid sequences 10, 11, 20 and 21 of WO2022058445A1, EM801 was prepared according to the amino acid sequences 43, 44, 45 and 46 of WO2016020332A1. The data was fitted in BIAcore 8K's evaluation software, and K_{D} values of the test antibodies to the above antigens were determined and summarized in Table 1.

It can be seen from Table 1 that MBS314 had potent binding affinity to human CD3, human GPRC5D and human BCMA. MBS314 showed lower binding affinity to the CD3D&E complex than EM801 and JNJ.

**Table 1. Binding affinity of Antibody MBS314 to human GPRC5D, human BCMA and human CD3D&E**

| Antibody | Antigen | ka (1/Ms) | kd (1/s) | K_{D} (M) |
|---|---|---|---|---|
| JNJ | human GPRC5D | 9.30E+05 | 3.92E-03 | 4.21E-09 |
| MBS314 | human GPRC5D | 4.93E+05 | 1.74E-03 | 3.53E-09 |
| MBS314 | human BCMA | 6.42E+05 | 2.83E-04 | 4.41E-10 |
| EM801 | human BCMA | 2.28E+06 | 2.27E-03 | 9.96E-10 |
| MBS314 | human CD3D&E | 3.11E+04 | 7.74E-03 | 2.49E-07 |
| JNJ | human CD3D&E | 4.44E+05 | 1.14E-02 | 2.56E-08 |
| EM801 | human CD3D&E | 1.85E+05 | 7.57E-03 | 4.10E-08 |

### Example 5. Binding capability of tri-specific CD3×BCMA×GPRC5D antibody to HEK293A/human BCMA cells, HEK293A/monkey BCMA cells, HEK293A/mouse BCMA cells, HEK293T/human GPRC5D cells, HEK293T/monkey GPRC5D cells and Jurkat cells

The binding activity of MBS314 to human BCMA, monkey BCMA, mouse BCMA, human GPRC5D, monkey GPRC5D and human CD3 complex expressed on cell surfaces were measured by FACS.

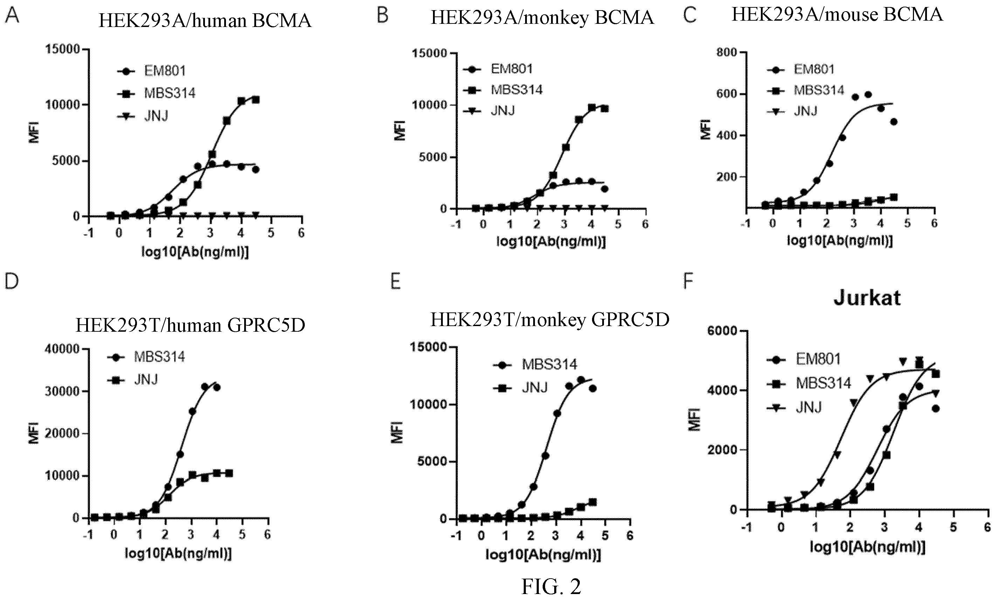

Specifically, the HEK293A/human BCMA cells, HEK293A/monkey BCMA cells, HEK293A/mouse BCMA cells, HEK293T/human GPRC5D cells, HEK293T/monkey GPRC5D cells (prepared in Example 1) and Jurkat cells (Nanjing Cobioer) with good growth status were digested by trypsin, collected, and centrifuged at 300 g for 5 min to remove the culture medium. The cells were re-suspended in PBS, counted, and centrifuged again to remove the supernatants. The cells were re-suspended in 2% FBS-PBS, to adjust the cell concentration to 4×10⁶/ml. Each kind of cells were added to 96-well U-bottom plates, 50 µl/well. The antibodies were diluted in 2% FBS-PBS, 5-fold serial dilution starting at 40 µg/ml, obtaining 12 concentrations in total. The 96-well plates were added with 50 µl antibody dilution, mixed well, and incubated at room temperature for 1 h. The plates were washed by 2% FBS-PBS, 200 µl/well, for three times. The plates were added with PE labelled goat-anti-human IgG (H+L) secondary antibody (1:500 diluted), 50 µl/well, and incubated at room temperature for 45 min. The plates were washed by 2% FBS-PBS, 200 µl/well, for three times. Finally the plates were added with 150 µl 2% FBS-PBS to re-suspend the cells, and the cells were tested in a flow cytometer. The data was processed in FlowJo and analyzed in GraphPad. The test results were shown in FIG. 2.

As shown in FIG. 2 (A-C), the antibody MBS314 had potent binding ability to HEK293A/human BCMA cells and HEK293A/monkey BCMA cells, in a dose-dependent manner, but did not bind mouse BCMA. FIG. 2 (D and E) showed that the antibody MBS314 bound to HEK293T/human GPRC5D cells and HEK293T/monkey GPRC5D cells, and the binding activity of MBS314 to human GPRC5D and monkey GPRC5D was significantly higher than that of JNJ. As shown in FIG. 2 (F), MBS314 had relatively low CD3 complex binding activity compared to JNJ.

### Example 6. Effect of tri-specific CD3xBCMAxGPRC5D antibody on activation of T cell signaling

The effect of the tri-specific antibody of the disclosure on T cell signaling activation was tested as follows. When the antibodies bound GPRC5D and/or BCMA on tumor cell surfaces and were thus present in the "cross-linking" status, they were able to bind CD3 on the surface of JURKAT-NFAT-Luc cells and activate NFAT signals, such that the JURKAT-NFAT-Luc cells can secrete luciferases. The antibody-mediated T cell activation level can be determined by measuring the luciferase activity.

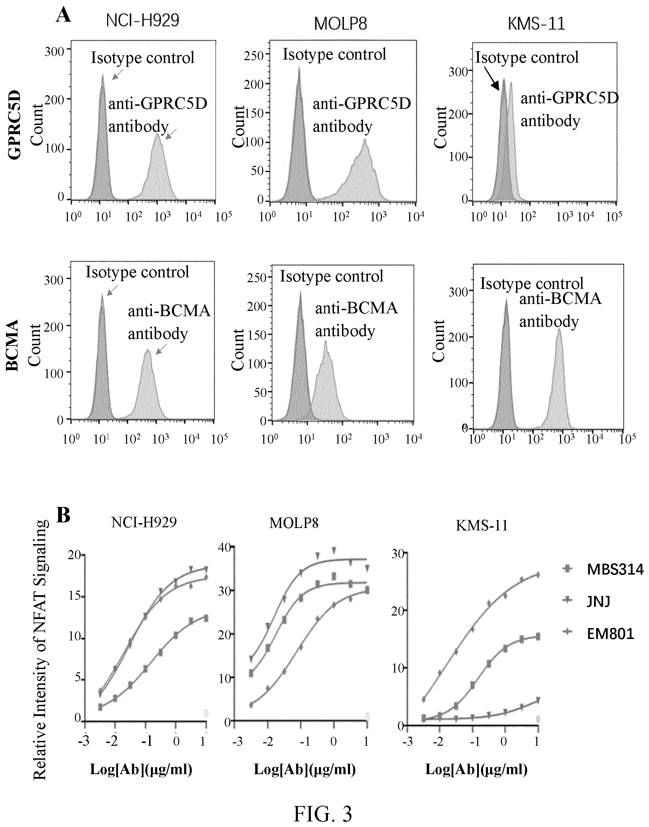

Firstly, the expression levels of GPRC5D and BCMA on the surface of multiple myeloma cells were measured using an anti-GPRC5D antibody and an anti-BCMA antibody produced by hybridoma technique (developed by KYINNO). The NCI-H929 cells with high GPRC5D and BCMA expression (KC-0629, KYINNO), MOLP8 cells with high GPRC5D expression but low BCMA expression (KC-0622, KYINNO) and KMS-11 cells with low GPRC5D cells but high BCMA expression (KC-0611, KYINNO) were picked for the subsequent T cell activation assay. Specifically, the myeloma cells (1 billion/ml, 100,000 cells/well) were suspended in 100 µl PBS (containing 2% BSA), and incubated with the anti-GPRC5D antibody or anti-BCMA antibody (produced by hybridoma approach, at 20 µg/ml) at room temperature for half an hour. After centrifugation and washing, the cells were added with PE-anti-mouse secondary antibody (1:400, Biolegend, 405307). After centrifugation and washing, the cells were suspended, added with 7-AAD, and measured for the GPRC5D and BCMA expression levels on tumor cells by flow cytometry. FIG. 3 (A) showed the flow cytometry analysis graphs of the three cells.

The myeloma cells, i.e., NCI-H929, MOLP8 and KSM-11 cells, and the JURKAT-NFAT-LUC cells (Cat#: KC-1485, KYINNO), were harvested and centrifuged at 400 g for 5 min, and the supematants were discarded. The cell density was adjusted to ~5×10⁵/ml using the culture medium (RPMI1640 containing 10% FBS). In the meanwhile, the antibodies were subjected to 3.16-fold serial dilution with the culture medium, starting at 10 µg/ml. The cells were evenly distributed, and 45 µl JURKAT-NFAT-Luc cells with 45 µl H929, MOLP8 or KSM-11 cells were added to 96-well U-bottom plates. The plates were added with 10 µl antibodies at various concentrations, and incubated in a 37°C incubator for 4-6 hr. Then, the cells were transferred to 96-well clear flat-bottom black-wall plates, added with Bright-Glo solution (E2610, promega), and read for the luminescence in a microplate reader in the multi-mode, for measuring the luciferase signals. The test results were shown in FIG. 3 (B), MBS314 activated CD3 signaling in all three cells, even if the GPRC5D or BCMA expression level on the tumor cells was relatively low. MBS314 induced significantly lower CD3 signaling in tumor cells with high GPRC5D and BCMA expressions than JNJ and EM801.

### Example 7. In vivo anti-tumor efficacy of tri-specific CD3xBCMAxGPRC5D antibody

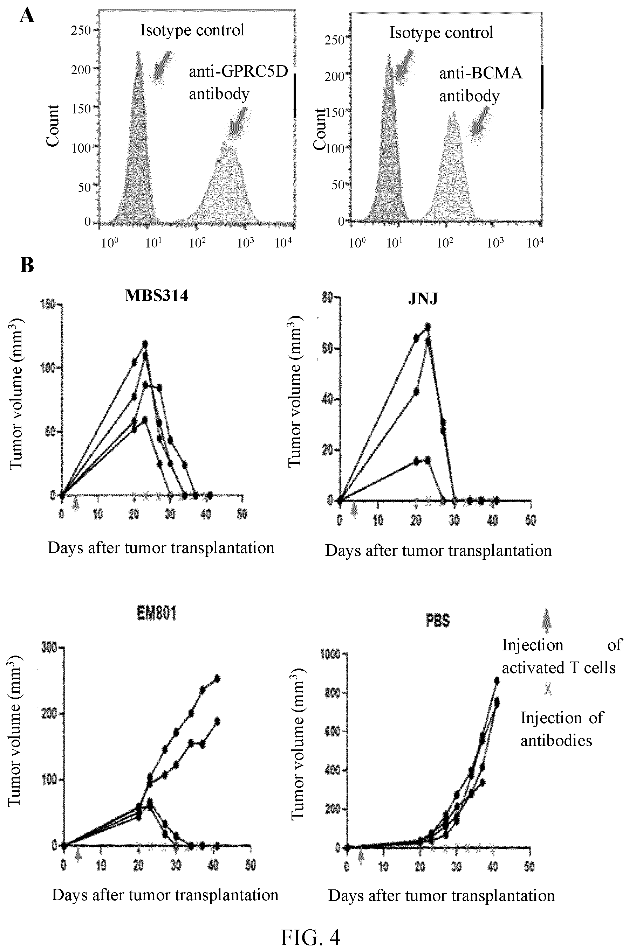

The MM.1S myeloma cells (KC-0620, KYINNO) expressing both GPRC5D and BCMA were subcutaneously injected into immune-deficient B-NDG mice (BIOTYTOGEN), and these mice were also intravenously injected with human T cells for establishing a humanized mouse model for *in vivo* anti-tumor efficacy. FIG. 4 (A) showed the GPRC5D and BCMA expression on MM.1S cells.

About 2-3 weeks before the tumor cell inoculation, the MM.1S myeloma cells were resuscitated, and cultured in RPMI-1640 medium (containing 10%FBS), with cell passaging performed every 3 to 4 days. Six days before tumor cell inoculation, ~1×10⁸ PBMCs were resuscitated, and 1×10⁶ such cells were co-cultured with 20 µl CD3/CD28 immuno-beads (Cat#: 11161D, GIBCO) for about 8 days, for T cell expansion *in vitro.*

On Day 0, MM.1S tumor cells were collected, and the tumor cell density was adjusted to 2.5×10⁷/ml using a 1:1 mixture of matrigel (356234, GIBCO) and PBS. Each NDG mouse was subcutaneously injected with 0.2 ml of such mixture, i.e., 0.5×10⁷ cells/mouse, at the right side of abdomen. On Day 2, the expanded T cells were harvested and suspended in PBS containing 0.1% FBS and 10 ng/ml IL-2, at the density of about 0.5×10⁸/ml. Each mouse was injected, via tail vein, with 0.2 ml T cells, i.e., about 1×10⁷ cells. When tumors size reached 50-100 mm³, i.e., on Day 20, the tri-specific antibody of the disclosure, JNJ, EM801 or PBS were injected via tail vein, at the dose of 10 µg per mouse, two doses per week, for 7 doses in total.

Tumor sizes were measured every 3 days, till Day 40, and the tumor volume was calculated as 0.5×length×width².

FIG. 4 (B) showed the tumor size change in mouse individuals from each group. It can be seen that MBS314 was able to eliminate tumor cells in the mice, with the effect evidently better than that of EM801 but comparable to that of JNJ.

### Example 8. Lethal effect of tri-specific CD3xBCMAxGPRC5D antibody on clinical tumor samples

Bone marrow monocytes were isolated from fresh samples of patients with relapsed and refractory multiple myeloma, and suspended in RPMI1640 medium (containing 10% FBS, 1% penicillin-streptomycin, 100 ng/ml IL-6, and 10 ng/ml IL-2) at the density of 5×10⁵/ml. The antibodies were diluted by the culture medium, 10-fold serial dilution, starting at 100 µg/ml, obtaining 6 concentrations in total. The 96-well U-bottom plates were added with 90 µl/well cell suspension and 10 µl diluted antibodies at various concentrations. After 48-hr culture, the plates were centrifuged to remove supernatants, and the cells were re-suspended in 100 µl FITC-anti-human CD138 (1:100, Biolegend, 356508), and incubated on ice for 30 min. The plates were centrifuged to remove the unbound anti-CD138 antibodies, and the cells were added with 7-AAD and subjected to FACS to analyze the ratio of CD138⁺ cells to viable cells.

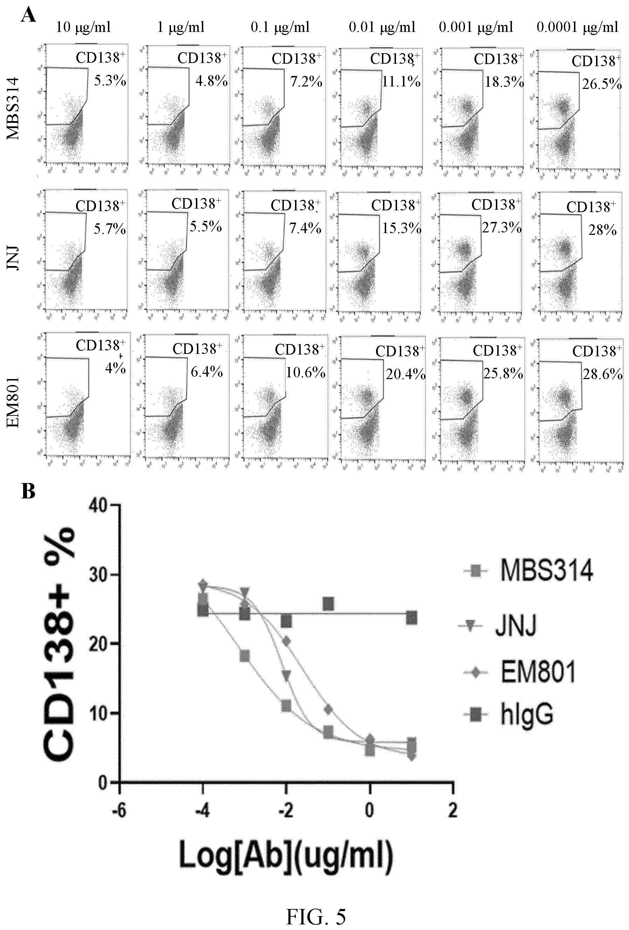

FIG. 5 (A) showed the FACS analysis graph, and FIG. 5 (B) was a statistics chart. The results indicated that the lethal effect of MBS314 on tumor cells was higher than those of JNJ and EM801.

### Example 9. In vivo toxicity of tri-specific CD3×BCMA×GPRC5D antibody

The tri-specific CD3×BCMA×GPRC5D antibody of the disclosure was tested for its *in vivo* toxic side effects in wild-type or CD3-humanized mice.

Particularly, 9 B-hCD3EDG mice (CD3-humanized or with human CD3, BIOTYTOGEN) were allocated into 3 groups, and 9 C57BL/6 mice were also allocated into 3 groups. These mice were injected via tail vein with MBS314, JNJ and PBS, respectively, at 20 mg/kg, 2 doses per week, 3 doses in total. The body weights were measured every day, and the ratio of body weight on that day to that of Day 0 was calculated, to plot the body weight curves during the test. On Day 10, the end of the test, 50-100 µl blood was collected from each of the wild-type or CD3-humanized mice, added with the anticoagulant, and subjected to routine blood tests. The heart, liver, spleen, kidney, bone marrow, brain, lung, and thymus were collected, and a pancreas cube with the edge length of 0.3-0.5 cm or shorter was cut using a blade, which were fixed in 10% neutral buffered formalin solution with a volume 10 times or more larger than that of the tissue, embedded in paraffin, sectioned, and stained with HE to observe tissue damage.

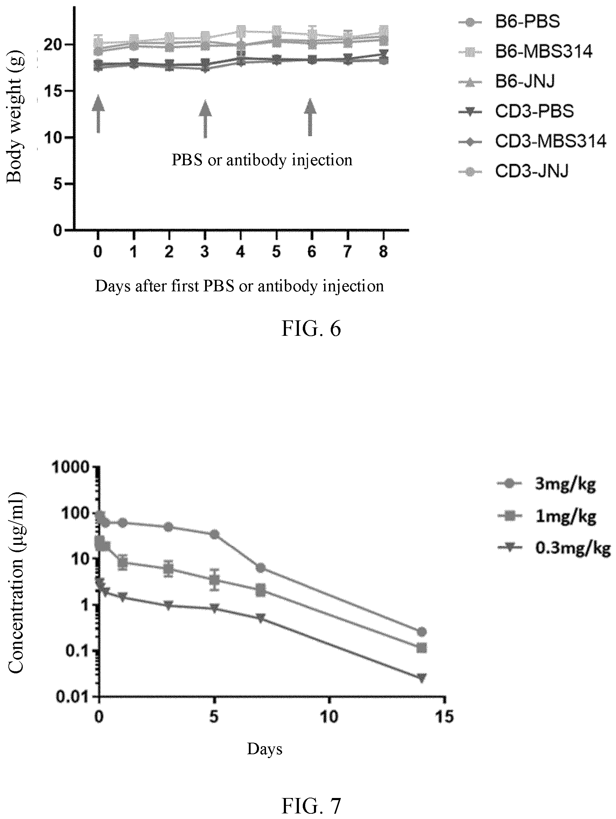

According to the test results, no evident body weight decrease (FIG. 6) or obvious tissue damage (results not shown) was observed in the wild-type mice and CD3-humniazed mice after being injected with MBS314 at a high dose, indicating that MBS314 had no obvious toxic side effects.

### Example 10. Pharmacokinetics (PK) of tri-specific CD3xBCMAxGPRC5D antibody in mice

Female BALB/c mice, 10-12-week old, were injected via tail vein with MBS314 at 3.0 mg/kg, 1.0 mg/kg or 0.3 mg/kg, and blood samples were collected after 10 min, 1 hr, 6 hr, 1 day, 3 days, 5 days, 7 days and 14 days. Particularly, the blood samples were collected from the retro-orbital sinus or the tail vein (added with anticoagulant after sampling), 0.03 ml per mouse each time. The blood samples at 10-30 µl were added with PBS of a volume 4 times larger than the blood sample, centrifuged at 10000 g for 5 min, collected for the supernatants, and stored at -20°C for subsequent uses.

The ELISA plates were added and coated with 50 ng/well BCMA-mFc (prepared by KYINNO) at 4°C overnight. On the 2^{nd} day, the plates were added with 200 µl blocking solution (2% BSA), and incubated at 37°C for 2 hr. The plasma samples as prepared above were respectively 1:50, 1: 100, and 1:500 diluted with PBS containing 2% BSA, and MBS314 was serially diluted with PBS containing 2% BSA, 3.16-fold serial dilution starting at 1 µg/ml, obtaining 12 concentrations in total, for preparation of the standard curve. The ELISA plates were added with 50 µl plasma sample and 50 µl MBS314, and incubated at 37°C for 1 hr. After washing, the plates were added with 50 µl HRP-anti-human Fc (1:10000, Solarbio, SE101), and incubated at 37°C for 1 hr. The plates were added with the ELISA color developing solution, 50 µl per well, color developed, and read for absorbance. The antibody concentration in the plasma was determined according to the standard curve and the plasma dilution factor.

FIG. 7 showed the antibody concentration-time curve, wherein the half-life of MBS314 in mouse blood was about 4.7 days when injected at 3.0 mg/kg, about 3.18 days when injected at 1.0 mg/kg, and about 4.97 days when injected at 0.3 mg/kg. The results indicated that the serum half-life of MBS314 in mice was about 4 to 5 days.

Exemplary sequences in the present application are set forth below.

| Description |
|---|
| Sequence/SEQ ID NO |
| VH-CDR1 of anti-BCMA antibody |
| NHIIH (SEQ ID NO: 1) |
| VH-CDR2 of anti-BCMA antibody |
| YINPYPGYHGYNDKFSG (SEQ ID NO: 2) |
| VH-CDR3 of anti-BCMA antibody |
| DGYYRDQDVLDY (SEQ ID NO: 3) |
| VL-CDR1 of anti-BCMA antibody |
| KASQDISQYLN (SEQ ID NO: 4) |
| VL-CDR2 of anti-BCMA antibody |
| YTSGLHP (SEQ ID NO: 5) |
| VL-CDR3 of anti-BCMA antibody |
| QQGYALPWT (SEQ ID NO: 6) |
| VH of anti-BCMA antibody |
| QVQLVQSGAEVKKPGASVKVSCKASGYTFTNHIIHWVRQAPGQGLEWMGYINPYPGYHGYND KFSGRATMTSDTSTSTVYMELSSLRSEDTAVYYCARDGYYRDQDVLDYWGQGTLVTVSS (SEQ ID NO: 7) |
| VL of anti-BCMA antibody |
| DIQMTQSPSSLSASVGDRVTITCKASQDISQYLNWYQQKPGKAPKLLIYYTSGLHPGVPSRFSGS GSGTDFTFTISSLEPEDEATYYCQQGYALPWTFGQGTKLTAK (SEQ ID NO: 8) |
| VH-CDR1 of anti-CD3 antibody |
| DFAMH (SEQ ID NO: 9) |
| VH-CDR2 of anti-CD3 antibody |
| GITWNSGSVGYADSVKG (SEQ ID NO: 10) |
| VH-CDR3 of anti-CD3 antibody |
| DRSGYGHYYYGMDV (SEQ ID NO: 11) |
| VL-CDR1 of anti-CD3 antibody |
| RASTSVSHNVA (SEQ ID NO: 12) |
| VL-CDR2 of anti-CD3 antibody |
| GASTKVT (SEQ ID NO: 13) |
| VL-CDR3 of anti-CD3 antibody |
| QHYINWPLT (SEQ ID NO: 14) |
| VH of anti-CD3 antibody |
| EVQLVESGGGLVQPGRSLRLSCAASGFTFDDFAMHWVRQAPGKGLEWVSGITWNSGSVGYAD SVKGRFTISRDNAKKSLYLQMNSLRAEDTALYYCAKDRSGYGHYYYGMDVWGQGTTVTVAS (SEQ ID NO: 15) |
| VL of anti-CD3 antibody |
| EIVMTQSPATLSVSPGERATLSCRASTSVSHNVAWYQQKPGQAPRLLIYGASTKVTGIPARFSGS GSGTEFTLTISSLQSEDFAVYYCQHYINWPLTFGGGTKVEIK (SEQ ID NO: 16) |
| VH-CDR1 of anti-GPRC5D antibody |
| SDYAWN (SEQ ID NO: 17) |
| VH-CDR2 of anti-GPRC5D antibody |
| YISYSGSATYSPSLKS (SEQ ID NO: 18) |
| VH-CDR3 of anti-GPRC5D antibody |
| GGIAGRGRWGAMDY (SEQ ID NO: 19) |
| VL-CDR1 of anti-GPRC5D antibody |
| KASQNVGTNVA (SEQ ID NO: 20) |
| VL-CDR2 of anti-GPRC5D antibody |
| SASYRDS (SEQ ID NO: 21) |
| VL-CDR3 of anti-GPRC5D antibody |
| QQYKSYPLT (SEQ ID NO: 22) |
| VH of anti-GPRC5D antibody |
| QVQLQESGPGLVKPSETLSLTCTVSGYSITSDYAWNWIRQPPGKGLEWMGYISYSGSATYSPSLK SRITISRDTSKNQFSLKLSSVTAADTAVYYCARGGIAGRGRWGAMDYWGQGTTVTVSS (SEQ ID NO: 23) |
| VL of anti-GPRC5D antibody |
| DIQMTQSPSSLSASVGDRVTITCKASQNVGTNVAWYQQKPGQSPKALIYSASYRDSGVPDRFSGS GSGTDFTLTISSLQPEDFATYYCQQYKSYPLTFGQGTRLEIK (SEQ ID NO: 24) |
| IgG1 Fc L234A/L235A/N297A/T366W in the long chain with knob mutation |
| ASTKGPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGGPSVFLFPPKPKD TLMISRTPEVTCVVVDVSHEDPEVKFNWYVDGVEVHNAKTKPREEQYASTYRVVSVLTVLHQD WLNGKEYKCKVSNKALPAPIEKTISKAKGQPREPQVYTLPPSREEMTKNQVSLX1CX2VKGFYPS DIAVEWESNGQPENNYKTTPPVLDSDGSFFLX3SKLTVDKSRWQQGNVFSCSVMHEALHNHYTQ KSLSLSPGK (SEQ ID NO: 25), X1=W, X2=L, X3=Y |
| |
| ASTKGPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGGPSVFLFPPKPKD TLMISRTPEVTCVVVDVSHEDPEVKFNWYVDGVEVHNAKTKPREEQYASTYRVVSVLTVLHQD WLNGKEYKCKVSNKALPAPIEKTISKAKGQPREPQVYTLPPSREEMTKNQVSLWCLVKGFYPSD IAVEWESNGQPENNYKTTPPVLDSDGSFFLYSKLTVDKSRWQQGNVFSCSVMHEALHNHYTQKS LSLSPGK |
| IgG1 Fc L234A/L235A/N297A/T366S/L368A/Y407V in the long chain with hole mutation |
| SEQ ID NO: 25, X1=S, X2=A, X3=V |
| ASTKGPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGGPSVFLFPPKPKD TLMISRTPEVTCVVVDVSHEDPEVKFNWYVDGVEVHNAKTKPREEQYASTYRVVSVLTVLHQD WLNGKEYKCKVSNKALPAPIEKTISKAKGQPREPQVYTLPPSREEMTKNQVSLSCAVKGFYPSDI AVEWESNGQPENNYKTTPPVLDSDGSFFLVSKLTVDKSRWQQGNVFSCSVMHEALHNHYTQKS LSLSPGK |
| κ light chain constant reigon |
|  (SEQ ID NO: 26) |
| Long chain of MBS314's knob-containing half-antibody |
| QVQLVQSGAEVKKPGASVKVSCKASGYTFTNHIIHWVRQAPGQGLEWMGYINPYPGYHGYND KFSGRATMTSDTSTSTVYMELSSLRSEDTAVYYCARDGYYRDQDVLDYWGQGTLVTVSSGGGG SGGGGSGGGGSGGGGSDIQMTQSPSSLSASVGDRVTITCKASQDISQYLNWYQQKPGKAPKLLI YYTSGLHPGVPSRFSGSGSGTDFTFTISSLEPEDEATYYCQQGYALPWTFGQGTKLTAKGGGGS GGGGSGGGGSEVQLVESGGGLVQPGRSLRLSCAASGFTFDDFAMHWVRQAPGKGLEWVSGIT WNSGSVGYADSVKGRFTISRDNAKKSLYLQMNSLRAEDTALYYCAKDRSGYGHYYYGMDVW GQGTTVTVASASTKGPSVFPLAPSSKSTSGGTAALGCLVKDYFPEPVTVSWNSGALTSGVHTFPAV |
| LQSSGLYSLSSVVTVPSSSLGTQTYICNVNHKPSNTKVDKKVEPKSCDKTHTCPPCPAPEAAGGPS VFLFPPKPKDTLMISRTPEVTCVVVDVSHEDPEVKFNWYVDGVEVHNAKTKPREEQYASTYRVV SVLTVLHQDWLNGKEYKCKVSNKALPAPIEKTISKAKGQPREPQVYTLPPSREEMTKNQVSLWC LVKGFYPSDIAVEWESNGQPENNYKTTPPVLDSDGSFFLYSKLTVDKSRWQQGNVFSCSVMHEA LHNHYTQKSLSLSPGK (SEQ ID NO: 27) |
| Short chain of MBS314's knob-containing half-antibody |
| EIVMTQSPATLSVSPGERATLSCRASTSVSHNVAWYQQKPGQAPRLLIYGASTKVTGIPARFSGSG SGTEFTLTISSLQSEDFAVYYCQHYINWPLTFGGGTKVEIKRTVAAPSVFIFPPSDEQLKSGTASVV CLLNNFYPREAKVQWKVDNALQSGNSQESVTEQDSKDSTYSLSSTLTLSKADYEKHKVYACEVT HQGLSSPVTKSFNRGEC (SEQ ID NO: 28) |
| Long chain of MBS314's hole-containing half-antibody |
| QVQLQESGPGLVKPSETLSLTCTVSGYSITSDYAWNWIRQPPGKGLEWMGYISYSGSATYSPSLK SRITISRDTSKNQFSLKLSSVTAADTAVYYCARGGIAGRGRWGAMDYWGQGTTVTVSSASTKG PSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCDKTHTCPPCPAPEAAGGPSVFLFPPKPKDTLMIS RTPEVTCVVVDVSHEDPEVKFNWYVDGVEVHNAKTKPREEQYASTYRVVSVLTVLHQDWLNG KEYKCKVSNKALPAPIEKTISKAKGQPREPQVYTLPPSREEMTKNQVSLSCAVKGFYPSDIAVEWE SNGQPENNYKTTPPVLDSDGSFFLVSKLTVDKSRWQQGNVFSCSVMHEALHNHYTQKSLSLSPG K (SEQ ID NO: 29) |
| Short chain of MBS314's hole-containing half-antibody |
| DIQMTQSPSSLSASVGDRVTITCKASQNVGTNVAWYQQKPGQSPKALIYSASYRDSGVPDRFSGS GSGTDFTLTISSLQPEDFATYYCQQYKSYPLTFGQGTRLEIKRTVAAPSVFIFPPSDEQLKSGTASV VCLLNNFYPREAKVQWKVDNALQSGNSQESVTEQDSKDSTYSLSSTLTLSKADYEKHKVYACE VTHQGLSSPVTKSFNRGEC (SEQ ID NO: 30) |
| Human BCMA |
| MLQMAGQCSQNEYFDSLLHACIPCQLRCSSNTPPLTCQRYCNASVTNSVKGTNAILWTCLGLSLII SLAVFVLMFLLRKINSEPLKDEFKNTGSGLLGMANIDLEKSRTGDEIILPRGLEYTVEECTCEDCIK SKPKVDSDHCFPLPAMEEGATILVTTKTNDYCKSLPAALSATEIEKSISAR (SEQ ID NO: 31) |
| Monkey BCMA |
| MLQMARQCSQNEYFDSLLHDCKPCQLRCSSTPPLTCQRYCNASMTNSVKGMNAILWTCLGLSLII SLAVFVLTFLLRKMSSEPLKDEFKNTGSGLLGMANIDLEKGRTGDEIVLPRGLEYTVEECTCEDCI KNKPKVDSDHCFPLPAMEEGATILVTTKTNDYCNSLSAALSVTEIEKSISAR (SEQ ID NO: 32) |
| Mouse BCMA |
| MAQQCFHSEYFDSLLHACKPCHLRCSNPPATCQPYCDPSVTSSVKGTYTVLWIFLGLTLVLSLALF TISFLLRKMNPEALKDEPQSPGQLDGSAQLDKADTELTRIRAGDDRIFPRSLEYTVEECTCEDCVK SKPKGDSDHFFPLPAMEEGATILVTTKTGDYGKSSVPTALQSVMGMEKPTHTR (SEQ ID NO: 33) |
| Human GPRC5D |
| MYKDCIESTGDYFLLCDAEGPWGIILESLAILGIVVTILLLLAFLFLMRKIQDCSQWNVLPTQLLFL LSVLGLFGLAFAFIIELNQQTAPVRYFLFGVLFALCFSCLLAHASNLVKLVRGCVSFSWTTILCIAIG CSLLQIIIATEYVTLIMTRGMMFVNMTPCQLNVDFVVLLVYVLFLMALTFFVSKATFCGPCENWK QHGRLIFITVLFSIIIWVVWISMLLRGNPQFQRQPQWDDPVVCIALVTNAWVFLLLYIVPELCILYR SCRQECPLQGNACPVTAYQHSFQVENQELSRARDSDGAEEDVALTSYGTPIQPQTVDPTQECFIPQ AKLSPQQDAGGV (SEQ ID NO: 34) |
| Monkey GPRC5D |
| MYKDCIESTGDYFLPCDSEGPWGIVLESLAILGIVVTILLLLAFLFLMRKIQDCSQWNVLPTQLLF LLSVLGLFGLAFAFIIQLNQQTAPVRYFLFGVLFALCFSCLLAHASNLVKLVRGRVSFSWTTILCIAI GCSLLQVIIAIEYVTLIMTRGMMFVHMTPYQLNVDFVVLLVYVLFLMALTFFVSKATFCGPCENW KQHGRLIFITVLFSIIIWVVWISMLLRGNPQFQRQPQWDDPVVCIALVTNAWVFLLLYIVPELCILY RSCRQECPSQGHACPVTAYQRSFQVENQELSRDC (SEQ ID NO: 35) |
| linker |
| GGGGSGGGGSGGGGS (SEQ ID NO: 36) |
| GGGGSGGGGSGGGGSGGGGS (SEQ ID NO: 37) |

Although the present application has been described in one or more embodiments, it is to be understood that the invention is not limited to these embodiments, and the description intends to cover all other alternatives, modifications and equivalents within the spirit and scope of the appended claims. All the references cited herein are incorporated by reference in their entirety.

## Claims

1. A multi-specific antibody, comprising:
i) an antigen binding domain against CD3,
ii) an antigen binding domain against BCMA, and
iii) an antigen binding domain against GPRC5D.

2. The multi-specific antibody of claim 1, comprising:
i) a first polypeptide chain, comprising a heavy chain variable region specifically binding BCMA, a light chain variable region specifically binding BCMA, a heavy chain variable region specifically binding CD3, and a heavy chain constant region,
ii) a second polypeptide chain, comprising a light chain variable region specifically binding CD3,
iii) a third polypeptide chain, comprising a heavy chain variable region specifically binding GPRC5D, and a heavy chain constant region, and
iv) a fourth polypeptide chain, comprising a light chain variable region specifically binding GPRC5D; or
i) a first polypeptide chain, comprising a heavy chain variable region specifically binding CD3, and a heavy chain constant region,
ii) a second polypeptide chain, comprising a heavy chain variable region specifically binding BCMA, a light chain variable region specifically binding BCMA, and a light chain variable region specifically binding CD3,
iii) a third polypeptide chain, comprising a heavy chain variable region specifically binding GPRC5D, and a heavy chain constant region, and
iv) a fourth polypeptide chain, comprising a light chain variable region specifically binding GPRC5D,
wherein the heavy chain variable region specifically binding BCMA and the light chain variable region specifically binding BCMA form the antigen binding domain against BCMA, the heavy chain variable region specifically binding CD3 in the first polypeptide chain and the light chain variable region specifically binding CD3 in the second polypeptide chain form the antigen binding domain against CD3, the heavy chain variable region specifically binding GPRC5D in the third polypeptide chain and the light chain variable region specifically binding GPRC5D in the fourth polypeptide chain form the antigen binding domain against GPRC5D, and the heavy chain constant region in the first polypeptide chain and the heavy chain constant region in the third polypeptide chain are associated together.

3. The multi-specific antibody of claim 2, wherein,
the first polypeptide chain comprises, from N-terminus to C-terminus, the heavy chain variable region specifically binding BCMA, the light chain variable region specifically binding BCMA, the heavy chain variable region specifically binding CD3, and the heavy chain constant region; or from N-terminus to C-terminus, the light chain variable region specifically binding BCMA, the heavy chain variable region specifically binding BCMA, the heavy chain variable region specifically binding CD3, and the heavy chain constant region, and
the third polypeptide chain comprises, from N-terminus to C-terminus, the heavy chain variable region specifically binding GPRC5D, and the heavy chain constant region; or
the first polypeptide chain comprises, from N-terminus to C-terminus, the heavy chain variable region specifically binding CD3, and the heavy chain constant region,
the second polypeptide chain comprises, from N-terminus to C-terminus, the heavy chain variable region specifically binding BCMA, the light chain variable region specifically binding BCMA, and the light chain variable region specifically binding CD3; or from N-terminus to C-terminus, the light chain variable region specifically binding BCMA, the heavy chain variable region specifically binding BCMA, and the light chain variable region specifically binding CD3, and
the third polypeptide chain comprises, from N-terminus to C-terminus, the heavy chain variable region specifically binding GPRC5D, and the heavy chain constant region.

4. The multi-specific antibody of claim 3, wherein,
i) the first polypeptide chain comprises, from N-terminus to C-terminus, the heavy chain variable region specifically binding BCMA, a first linker, the light chain variable region specifically binding BCMA, a second linker, the heavy chain variable region specifically binding CD3, and the heavy chain constant region; or the light chain variable region specifically binding BCMA, a first linker, the heavy chain variable region specifically binding BCMA, a second linker, the heavy chain variable region specifically binding CD3, and the heavy chain constant region,
ii) the second polypeptide chain comprises, from N-terminus to C-terminus, the light chain variable region specifically binding CD3 and a light chain constant region,
iii) the third polypeptide chain comprises, from N-terminus to C-terminus, the heavy chain variable region specifically binding GPRC5D, and the heavy chain constant region, and
iv) the fourth polypeptide chain comprises, from N-terminus to C-terminus, the light chain variable region specifically binding GPRC5D, and a light chain constant region; or
i) the first polypeptide chain comprises, from N-terminus to C-terminus, the heavy chain variable region specifically binding CD3, and the heavy chain constant region,
ii) the second polypeptide chain comprises, from N-terminus to C-terminus, the heavy chain variable region specifically binding BCMA, a first linker, the light chain variable region specifically binding BCMA, a second linker, the light chain variable region specifically binding CD3, and a light chain constant region; or the light chain variable region specifically binding BCMA, a first linker, the heavy chain variable region specifically binding BCMA, a second linker, the light chain variable region specifically binding CD3, and a light chain constant region,
iii) the third polypeptide chain comprises, from N-terminus to C-terminus, the heavy chain variable region specifically binding GPRC5D, and the heavy chain constant region, and
iv) the fourth polypeptide chain comprises, from N-terminus to C-terminus, the light chain variable region specifically binding GPRC5D and a light chain constant region.

5. The multi-specific antibody of claim 1, wherein,
the antigen binding domain against CD3 is an antibody or an antigen-binding portion thereof specifically binding CD3, wherein a heavy chain variable region thereof comprises a VH-CDR1, a VH-CDR2 and a VH-CDR3 of SEQ ID NOs: 9-11, respectively, and a light chain variable region thereof comprises a VL-CDR1, a VL-CDR2 and a VL-CDR3 of SEQ ID NOs: 12-14, respectively,
the antigen binding domain against BCMA is an antibody or an antigen-binding portion thereof specifically binding BCMA, wherein a heavy chain variable region thereof comprises a VH-CDR1, a VH-CDR2 and a VH-CDR3 of SEQ ID NOs: 1-3, respectively, and a light chain variable region thereof comprises a VL-CDR1, a VL-CDR2 and a VL-CDR3 of SEQ ID NOs: 4-6, respectively, and/or
the antigen binding domain against GPRC5D is an antibody or an antigen-binding portion thereof specifically binding GPRC5D, wherein a heavy chain variable region thereof comprises a VH-CDR1, a VH-CDR2 and a VH-CDR3 of SEQ ID NOs: 17-19, respectively, and a light chain variable region thereof comprises a VL-CDR1, a VL-CDR2 and a VL-CDR3 of SEQ ID NOs: 20-22, respectively.

6. The multi-specific antibody of claim 5, wherein,
the heavy chain variable region and the light chain variable region in the antigen binding domain against CD3 comprise amino acid sequences having at least 85%, 86%, 87%, 88%, 89%, 90%, 91%, 92%, 93%, 94%, 95%, 96%, 97%, 98%, 99% or 100% sequence identity to SEQ ID NOs: 15 and 16, respectively,
the heavy chain variable region and the light chain variable region in the antigen binding domain against BCMA comprise amino acid sequences having at least 85%, 86%, 87%, 88%, 89%, 90%, 91%, 92%, 93%, 94%, 95%, 96%, 97%, 98%, 99% or 100% sequence identity to SEQ ID NOs: 7 and 8, respectively, and/or
the heavy chain variable region and the light chain variable region in the antigen binding domain against GPRC5D comprise amino acid sequences having at least 85%, 86%, 87%, 88%, 89%, 90%, 91%, 92%, 93%, 94%, 95%, 96%, 97%, 98%, 99% or 100% sequence identity to SEQ ID NOs: 23 and 24, respectively.

7. The multi-specific antibody of claim 2, wherein the heavy chain constant region in the first polypeptide chain and the heavy chain constant region in the third polypeptide chain are human heavy chain constant region with weak or no binding to Fc receptors and/or complement system proteins, and/or
one of the heavy chain constant region in the first polypeptide chain and the heavy chain constant region in the third polypeptide chain is with a knob mutation, and the other is with a hole mutation.

8. The multi-specific antibody of claim 4, wherein the first linker and/or the second linker comprise(s) the amino acid sequence(s) of SEQ ID NOs: 36 and 37.

9. The multi-specific antibody of claim 2, wherein the first polypeptide chain, the second polypeptide chain, the third polypeptide chain and the fourth polypeptide chain comprise amino acid sequences having at least 85%, 86%, 87%, 88%, 89%, 90%, 91%, 92%, 93%, 94%, 95%, 96%, 97%, 98%, 99% or 100% sequence identity to SEQ ID NOs: 27, 28, 29 and 30, respectively.

10. A nucleic acid molecule, encoding the multi-specific antibody of any one of claims 1 to 9.

11. An expression vector, comprising the nucleic acid sequence of claim 10.

12. A host cell, comprising the nucleic acid molecule of claim 10, or the expression vector of claim 11.

13. A pharmaceutical composition, comprising the multi-specific antibody of any one of claims 1 to 9, the nucleic acid molecule of claim 10, the expression vector of claim 11, or the host cell of claim 12, and a pharmaceutically acceptable carrier.

14. Use of the pharmaceutical composition of claim 13 in preparation of a medicament for treating a BCMA and/or GPRC5D associated disease.

15. The use of claim 14, wherein the BCMA and/or GPRC5D associated disease is multiple myeloma.
